# EUROPEAN PATENT SPECIFICATION

(11) **EP 3 645 717 B1**
(45) Date of publication and mention of the grant of the patent: **11.08.2021**
(21) Application number: 18734550.9
(22) Date of filing: 27.06.2018
(51) Int. Cl.: C12N 15/10, C40B 40/06

(54) **MODULAR NUCLEIC ACID ADAPTERS**
MODULARE NUKLEINSÄUREADAPTER
ADAPTATEURS MODULAIRES D'ACIDE NUCLÉIQUE

(30) Priority: 27.06.2017 US 201762525595 P
(43) Date of publication of application: 06.05.2020
(73) Proprietor: F. Hoffmann-La Roche AG, 4070 Basel (CH); Roche Diagnostics GmbH, 68305 Mannheim (DE)
(72) Inventor: KLASS, Daniel, Pleasanton California 94588 (US); LOVEJOY, Alexander, Pleasanton California 94588 (US); MIREBRAHIM, Seyed Hamid, Pleasanton California 94588 (US); PATI, Amrita, Pleasanton California 94588 (US)
(74) Representative: Hildebrandt, Martin K. E.
(86) International application number: PCT/EP2018/067246
(87) International publication number: WO 2019/002366

(56) References cited:
- WO-A1-2017/129647
- WO-A2-2008/093098
- AARON M NEWMAN ET AL: "Integrated digital error suppression for improved detection of circulating tumor DNA", NATURE BIOTECHNOLOGY, vol. 34, no. 5, 28 March 2016 (2016-03-28) , pages 547-555, XP055464348, ISSN: 1087-0156, DOI: 10.1038/nbt.3520
- BRANT C. FAIRCLOTH ET AL: "Not All Sequence Tags Are Created Equal: Designing and Validating Sequence Identification Tags Robust to Indels", PLOS ONE, vol. 7, no. 8, 10 August 2012 (2012-08-10) , page e42543, XP055499431, DOI: 10.1371/journal.pone.0042543 cited in the application
- PRASHAR Y ET AL: "ANALYSIS OF DIFFERENTIAL GENE EXPRESSION BY DISPLAY OF 3' END RESTRICTION FRAGMENTS FO CDNAS", PROCEEDINGS OF THE NATIONAL ACADEMY OF SCIENCES, NATIONAL ACADEMY OF SCIENCES, US, vol. 93, 1 January 1996 (1996-01-01), pages 659-663, XP002059941, ISSN: 0027-8424, DOI: 10.1073/PNAS.93.2.659

### SEQUENCE LISTING

<110> ROCHE SEQUENCING SOLUTIONS, INC.
   F. HOFFMANN-LA ROCHE AG
   ROCHE DIAGNOSTICS GMBH
<120> MODULAR NUCLEIC ACID ADAPTERS
<130> P34320-WO
<140>
   <141>
<150> 62/525,595
   <151> 2017-06-27
<160> 217
<170> PatentIn version 3.5
<210> 1
   <211> 36
   <212> DNA
   <213> Artificial Sequence
<220>
   <221> source
   <223> /note="Description of Artificial Sequence: Synthetic oligonucleotide"
<400> 1
   tctttcccta cacgacgctc ttccgatctc agatct 36
<210> 2
   <211> 34
   <212> DNA
   <213> Artificial Sequence
<220>
   <221> source
   <223> /note="Description of Artificial Sequence: Synthetic oligonucleotide"
<400> 2
   agatcggaag agcacacgtc tgaactccag tcac 34
<210> 3
   <211> 36
   <212> DNA
   <213> Artificial Sequence
<220>
   <221> source
   <223> /note="Description of Artificial Sequence: Synthetic oligonucleotide"
<400> 3
   tctttcccta cacgacgctc ttccgatctc agatct 36
<210> 4
   <211> 40
   <212> DNA
   <213> Artificial Sequence
<220>
   <221> source
   <223> /note="Description of Artificial Sequence: Synthetic oligonucleotide"
<400> 4
   gatctgagat cggaagagca cacgtctgaa ctccagtcac 40
<210> 5
   <211> 62
   <212> DNA
   <213> Artificial Sequence
<220>
   <221> source
   <223> /note="Description of Artificial Sequence: Synthetic oligonucleotide"
<400> 5
   aatgatacgg cgaccaccga gatctacacg ttaagcgaca ctctttccct acacgacgct 60
   ct 62
<210> 6
   <211> 62
   <212> DNA
   <213> Artificial Sequence
<220>
   <221> source
   <223> /note="Description of Artificial Sequence: Synthetic oligonucleotide"
<400> 6
   aatgatacgg cgaccaccga gatctacacg agaccaaaca ctctttccct acacgacgct 60
   ct 62
<210> 7
   <211> 62
   <212> DNA
   <213> Artificial Sequence
<220>
   <221> source
   <223> /note="Description of Artificial Sequence: Synthetic oligonucleotide"
<400> 7
   aatgatacgg cgaccaccga gatctacaca gccgtaaaca ctctttccct acacgacgct 60
   ct 62
<210> 8
   <211> 62
   <212> DNA
   <213> Artificial Sequence
<220>
   <221> source
   <223> /note="Description of Artificial Sequence: Synthetic oligonucleotide"
<400> 8
   aatgatacgg cgaccaccga gatctacact tcgaagcaca ctctttccct acacgacgct 60
   ct 62
<210> 9
   <211> 62
   <212> DNA
   <213> Artificial Sequence
<220>
   <221> source
   <223> /note="Description of Artificial Sequence: Synthetic oligonucleotide"
<400> 9
   aatgatacgg cgaccaccga gatctacaca tgacaggaca ctctttccct acacgacgct 60
   ct 62
<210> 10
   <211> 62
   <212> DNA
   <213> Artificial Sequence
<220>
   <221> source
   <223> /note="Description of Artificial Sequence: Synthetic oligonucleotide"
<400> 10
   aatgatacgg cgaccaccga gatctacact cgtgcataca ctctttccct acacgacgct 60
   ct 62
<210> 11
   <211> 62
   <212> DNA
   <213> Artificial Sequence
<220>
   <221> source
   <223> /note="Description of Artificial Sequence: Synthetic oligonucleotide"
<400> 11
   aatgatacgg cgaccaccga gatctacacc gaagtcaaca ctctttccct acacgacgct 60
   ct 62
<210> 12
   <211> 62
   <212> DNA
   <213> Artificial Sequence
<220>
   <221> source
   <223> /note="Description of Artificial Sequence: Synthetic oligonucleotide"
<400> 12
   aatgatacgg cgaccaccga gatctacacg aatccgtaca ctctttccct acacgacgct 60
   ct 62
<210> 13
   <211> 62
   <212> DNA
   <213> Artificial Sequence
<220>
   <221> source
   <223> /note="Description of Artificial Sequence: Synthetic oligonucleotide"
<400> 13
   aatgatacgg cgaccaccga gatctacacg aagtgctaca ctctttccct acacgacgct 60
   ct 62
<210> 14
   <211> 62
   <212> DNA
   <213> Artificial Sequence
<220>
   <221> source
   <223> /note="Description of Artificial Sequence: Synthetic oligonucleotide"
<400> 14
   aatgatacgg cgaccaccga gatctacacg tccttgaaca ctctttccct acacgacgct 60
   ct 62
<210> 15
   <211> 62
   <212> DNA
   <213> Artificial Sequence
<220>
   <221> source
   <223> /note="Description of Artificial Sequence: Synthetic oligonucleotide"
<400> 15
   aatgatacgg cgaccaccga gatctacacc atgtgtgaca ctctttccct acacgacgct 60
   ct 62
<210> 16
   <211> 62
   <212> DNA
   <213> Artificial Sequence
<220>
   <221> source
   <223> /note="Description of Artificial Sequence: Synthetic oligonucleotide"
<400> 16
   aatgatacgg cgaccaccga gatctacaca cctcttcaca ctctttccct acacgacgct 60
   ct 62
<210> 17
   <211> 62
   <212> DNA
   <213> Artificial Sequence
<220>
   <221> source
   <223> /note="Description of Artificial Sequence: Synthetic oligonucleotide"
<400> 17
   aatgatacgg cgaccaccga gatctacact ccgatcaaca ctctttccct acacgacgct 60
   ct 62
<210> 18
   <211> 62
   <212> DNA
   <213> Artificial Sequence
<220>
   <221> source
   <223> /note="Description of Artificial Sequence: Synthetic oligonucleotide"
<400> 18
   aatgatacgg cgaccaccga gatctacacc gtatctcaca ctctttccct acacgacgct 60
   ct 62
<210> 19
   <211> 62
   <212> DNA
   <213> Artificial Sequence
<220>
   <221> source
   <223> /note="Description of Artificial Sequence: Synthetic oligonucleotide"
<400> 19
   aatgatacgg cgaccaccga gatctacact tgcaacgaca ctctttccct acacgacgct 60
   ct 62
<210> 20
   <211> 62
   <212> DNA
   <213> Artificial Sequence
<220>
   <221> source
   <223> /note="Description of Artificial Sequence: Synthetic oligonucleotide"
<400> 20
   aatgatacgg cgaccaccga gatctacact gataggcaca ctctttccct acacgacgct 60
   ct 62
<210> 21
   <211> 62
   <212> DNA
   <213> Artificial Sequence
<220>
   <221> source
   <223> /note="Description of Artificial Sequence: Synthetic oligonucleotide"
<400> 21
   aatgatacgg cgaccaccga gatctacaca acagtccaca ctctttccct acacgacgct 60
   ct 62
<210> 22
   <211> 62
   <212> DNA
   <213> Artificial Sequence
<220>
   <221> source
   <223> /note="Description of Artificial Sequence: Synthetic oligonucleotide"
<400> 22
   aatgatacgg cgaccaccga gatctacaca ggaacacaca ctctttccct acacgacgct 60
   ct 62
<210> 23
   <211> 62
   <212> DNA
   <213> Artificial Sequence
<220>
   <221> source
   <223> /note="Description of Artificial Sequence: Synthetic oligonucleotide"
<400> 23
   aatgatacgg cgaccaccga gatctacact cctcatgaca ctctttccct acacgacgct 60
   ct 62
<210> 24
   <211> 62
   <212> DNA
   <213> Artificial Sequence
<220>
   <221> source
   <223> /note="Description of Artificial Sequence: Synthetic oligonucleotide"
<400> 24
   aatgatacgg cgaccaccga gatctacaca gagcagaaca ctctttccct acacgacgct 60
   ct 62
<210> 25
   <211> 62
   <212> DNA
   <213> Artificial Sequence
<220>
   <221> source
   <223> /note="Description of Artificial Sequence: Synthetic oligonucleotide"
<400> 25
   aatgatacgg cgaccaccga gatctacacg aacgaagaca ctctttccct acacgacgct 60
   ct 62
<210> 26
   <211> 62
   <212> DNA
   <213> Artificial Sequence
<220>
   <221> source
   <223> /note="Description of Artificial Sequence: Synthetic oligonucleotide"
<400> 26
   aatgatacgg cgaccaccga gatctacact tgagctcaca ctctttccct acacgacgct 60
   ct 62
<210> 27
   <211> 62
   <212> DNA
   <213> Artificial Sequence
<220>
   <221> source
   <223> /note="Description of Artificial Sequence: Synthetic oligonucleotide"
<400> 27
   aatgatacgg cgaccaccga gatctacacg ctgaatcaca ctctttccct acacgacgct 60
   ct 62
<210> 28
   <211> 62
   <212> DNA
   <213> Artificial Sequence
<220>
   <221> source
   <223> /note="Description of Artificial Sequence: Synthetic oligonucleotide"
<400> 28
   aatgatacgg cgaccaccga gatctacaca gattgcgaca ctctttccct acacgacgct 60
   ct 62
<210> 29
   <211> 62
   <212> DNA
   <213> Artificial Sequence
<220>
   <221> source
   <223> /note="Description of Artificial Sequence: Synthetic oligonucleotide"
<400> 29
   aatgatacgg cgaccaccga gatctacacc aacttggaca ctctttccct acacgacgct 60
   ct 62
<210> 30
   <211> 62
   <212> DNA
   <213> Artificial Sequence
<220>
   <221> source
   <223> /note="Description of Artificial Sequence: Synthetic oligonucleotide"
<400> 30
   aatgatacgg cgaccaccga gatctacact tggtgcaaca ctctttccct acacgacgct 60
   ct 62
<210> 31
   <211> 62
   <212> DNA
   <213> Artificial Sequence
<220>
   <221> source
   <223> /note="Description of Artificial Sequence: Synthetic oligonucleotide"
<400> 31
   aatgatacgg cgaccaccga gatctacacc tgtaccaaca ctctttccct acacgacgct 60
   ct 62
<210> 32
   <211> 62
   <212> DNA
   <213> Artificial Sequence
<220>
   <221> source
   <223> /note="Description of Artificial Sequence: Synthetic oligonucleotide"
<400> 32
   aatgatacgg cgaccaccga gatctacaca ctctgagaca ctctttccct acacgacgct 60
   ct 62
<210> 33
   <211> 62
   <212> DNA
   <213> Artificial Sequence
<220>
   <221> source
   <223> /note="Description of Artificial Sequence: Synthetic oligonucleotide"
<400> 33
   aatgatacgg cgaccaccga gatctacacc tcctagtaca ctctttccct acacgacgct 60
   ct 62
<210> 34
   <211> 62
   <212> DNA
   <213> Artificial Sequence
<220>
   <221> source
   <223> /note="Description of Artificial Sequence: Synthetic oligonucleotide"
<400> 34
   aatgatacgg cgaccaccga gatctacacg ccaatacaca ctctttccct acacgacgct 60
   ct 62
<210> 35
   <211> 62
   <212> DNA
   <213> Artificial Sequence
<220>
   <221> source
   <223> /note="Description of Artificial Sequence: Synthetic oligonucleotide"
<400> 35
   aatgatacgg cgaccaccga gatctacacc ctcatctaca ctctttccct acacgacgct 60
   ct 62
<210> 36
   <211> 62
   <212> DNA
   <213> Artificial Sequence
<220>
   <221> source
   <223> /note="Description of Artificial Sequence: Synthetic oligonucleotide"
<400> 36
   aatgatacgg cgaccaccga gatctacact gagctgtaca ctctttccct acacgacgct 60
   ct 62
<210> 37
   <211> 62
   <212> DNA
   <213> Artificial Sequence
<220>
   <221> source
   <223> /note="Description of Artificial Sequence: Synthetic oligonucleotide"
<400> 37
   aatgatacgg cgaccaccga gatctacacg tctcatcaca ctctttccct acacgacgct 60
   ct 62
<210> 38
   <211> 62
   <212> DNA
   <213> Artificial Sequence
<220>
   <221> source
   <223> /note="Description of Artificial Sequence: Synthetic oligonucleotide"
<400> 38
   aatgatacgg cgaccaccga gatctacact aagcgcaaca ctctttccct acacgacgct 60
   ct 62
<210> 39
   <211> 62
   <212> DNA
   <213> Artificial Sequence
<220>
   <221> source
   <223> /note="Description of Artificial Sequence: Synthetic oligonucleotide"
<400> 39
   aatgatacgg cgaccaccga gatctacaca gctaccaaca ctctttccct acacgacgct 60
   ct 62
<210> 40
   <211> 62
   <212> DNA
   <213> Artificial Sequence
<220>
   <221> source
   <223> /note="Description of Artificial Sequence: Synthetic oligonucleotide"
<400> 40
   aatgatacgg cgaccaccga gatctacacc ttcactgaca ctctttccct acacgacgct 60
   ct 62
<210> 41
   <211> 62
   <212> DNA
   <213> Artificial Sequence
<220>
   <221> source
   <223> /note="Description of Artificial Sequence: Synthetic oligonucleotide"
<400> 41
   aatgatacgg cgaccaccga gatctacacg agagtacaca ctctttccct acacgacgct 60
   ct 62
<210> 42
   <211> 62
   <212> DNA
   <213> Artificial Sequence
<220>
   <221> source
   <223> /note="Description of Artificial Sequence: Synthetic oligonucleotide"
<400> 42
   aatgatacgg cgaccaccga gatctacacg cgttagaaca ctctttccct acacgacgct 60
   ct 62
<210> 43
   <211> 62
   <212> DNA
   <213> Artificial Sequence
<220>
   <221> source
   <223> /note="Description of Artificial Sequence: Synthetic oligonucleotide"
<400> 43
   aatgatacgg cgaccaccga gatctacaca ggcaatgaca ctctttccct acacgacgct 60
   ct 62
<210> 44
   <211> 62
   <212> DNA
   <213> Artificial Sequence
<220>
   <221> source
   <223> /note="Description of Artificial Sequence: Synthetic oligonucleotide"
<400> 44
   aatgatacgg cgaccaccga gatctacacg ctacaacaca ctctttccct acacgacgct 60
   ct 62
<210> 45
   <211> 62
   <212> DNA
   <213> Artificial Sequence
<220>
   <221> source
   <223> /note="Description of Artificial Sequence: Synthetic oligonucleotide"
<400> 45
   aatgatacgg cgaccaccga gatctacact cagtaggaca ctctttccct acacgacgct 60
   ct 62
<210> 46
   <211> 62
   <212> DNA
   <213> Artificial Sequence
<220>
   <221> source
   <223> /note="Description of Artificial Sequence: Synthetic oligonucleotide"
<400> 46
   aatgatacgg cgaccaccga gatctacacc tatgcctaca ctctttccct acacgacgct 60
   ct 62
<210> 47
   <211> 62
   <212> DNA
   <213> Artificial Sequence
<220>
   <221> source
   <223> /note="Description of Artificial Sequence: Synthetic oligonucleotide"
<400> 47
   aatgatacgg cgaccaccga gatctacact gctgtgaaca ctctttccct acacgacgct 60
   ct 62
<210> 48
   <211> 62
   <212> DNA
   <213> Artificial Sequence
<220>
   <221> source
   <223> /note="Description of Artificial Sequence: Synthetic oligonucleotide"
<400> 48
   aatgatacgg cgaccaccga gatctacacc cgaagataca ctctttccct acacgacgct 60
   ct 62
<210> 49
   <211> 62
   <212> DNA
   <213> Artificial Sequence
<220>
   <221> source
   <223> /note="Description of Artificial Sequence: Synthetic oligonucleotide"
<400> 49
   aatgatacgg cgaccaccga gatctacaca gaccttgaca ctctttccct acacgacgct 60
   ct 62
<210> 50
   <211> 62
   <212> DNA
   <213> Artificial Sequence
<220>
   <221> source
   <223> /note="Description of Artificial Sequence: Synthetic oligonucleotide"
<400> 50
   aatgatacgg cgaccaccga gatctacaca ctgcttgaca ctctttccct acacgacgct 60
   ct 62
<210> 51
   <211> 62
   <212> DNA
   <213> Artificial Sequence
<220>
   <221> source
   <223> /note="Description of Artificial Sequence: Synthetic oligonucleotide"
<400> 51
   aatgatacgg cgaccaccga gatctacact aagtggcaca ctctttccct acacgacgct 60
   ct 62
<210> 52
   <211> 62
   <212> DNA
   <213> Artificial Sequence
<220>
   <221> source
   <223> /note="Description of Artificial Sequence: Synthetic oligonucleotide"
<400> 52
   aatgatacgg cgaccaccga gatctacacc gcaatgtaca ctctttccct acacgacgct 60
   ct 62
<210> 53
   <211> 62
   <212> DNA
   <213> Artificial Sequence
<220>
   <221> source
   <223> /note="Description of Artificial Sequence: Synthetic oligonucleotide"
<400> 53
   aatgatacgg cgaccaccga gatctacact gaccgttaca ctctttccct acacgacgct 60
   ct 62
<210> 54
   <211> 62
   <212> DNA
   <213> Artificial Sequence
<220>
   <221> source
   <223> /note="Description of Artificial Sequence: Synthetic oligonucleotide"
<400> 54
   aatgatacgg cgaccaccga gatctacacc ctcgaataca ctctttccct acacgacgct 60
   ct 62
<210> 55
   <211> 62
   <212> DNA
   <213> Artificial Sequence
<220>
   <221> source
   <223> /note="Description of Artificial Sequence: Synthetic oligonucleotide"
<400> 55
   aatgatacgg cgaccaccga gatctacact gctctacaca ctctttccct acacgacgct 60
   ct 62
<210> 56
   <211> 62
   <212> DNA
   <213> Artificial Sequence
<220>
   <221> source
   <223> /note="Description of Artificial Sequence: Synthetic oligonucleotide"
<400> 56
   aatgatacgg cgaccaccga gatctacacg tcgttacaca ctctttccct acacgacgct 60
   ct 62
<210> 57
   <211> 62
   <212> DNA
   <213> Artificial Sequence
<220>
   <221> source
   <223> /note="Description of Artificial Sequence: Synthetic oligonucleotide"
<400> 57
   aatgatacgg cgaccaccga gatctacaca tagtcggaca ctctttccct acacgacgct 60
   ct 62
<210> 58
   <211> 62
   <212> DNA
   <213> Artificial Sequence
<220>
   <221> source
   <223> /note="Description of Artificial Sequence: Synthetic oligonucleotide"
<400> 58
   aatgatacgg cgaccaccga gatctacact agcaggaaca ctctttccct acacgacgct 60
   ct 62
<210> 59
   <211> 62
   <212> DNA
   <213> Artificial Sequence
<220>
   <221> source
   <223> /note="Description of Artificial Sequence: Synthetic oligonucleotide"
<400> 59
   aatgatacgg cgaccaccga gatctacact acggaagaca ctctttccct acacgacgct 60
   ct 62
<210> 60
   <211> 62
   <212> DNA
   <213> Artificial Sequence
<220>
   <221> source
   <223> /note="Description of Artificial Sequence: Synthetic oligonucleotide"
<400> 60
   aatgatacgg cgaccaccga gatctacaca ggtgttgaca ctctttccct acacgacgct 60
   ct 62
<210> 61
   <211> 62
   <212> DNA
   <213> Artificial Sequence
<220>
   <221> source
   <223> /note="Description of Artificial Sequence: Synthetic oligonucleotide"
<400> 61
   aatgatacgg cgaccaccga gatctacacc cgatgtaaca ctctttccct acacgacgct 60
   ct 62
<210> 62
   <211> 62
   <212> DNA
   <213> Artificial Sequence
<220>
   <221> source
   <223> /note="Description of Artificial Sequence: Synthetic oligonucleotide"
<400> 62
   aatgatacgg cgaccaccga gatctacacc tcgacttaca ctctttccct acacgacgct 60
   ct 62
<210> 63
   <211> 62
   <212> DNA
   <213> Artificial Sequence
<220>
   <221> source
   <223> /note="Description of Artificial Sequence: Synthetic oligonucleotide"
<400> 63
   aatgatacgg cgaccaccga gatctacacg tagtaccaca ctctttccct acacgacgct 60
   ct 62
<210> 64
   <211> 62
   <212> DNA
   <213> Artificial Sequence
<220>
   <221> source
   <223> /note="Description of Artificial Sequence: Synthetic oligonucleotide"
<400> 64
   aatgatacgg cgaccaccga gatctacaca ttagccgaca ctctttccct acacgacgct 60
   ct 62
<210> 65
   <211> 62
   <212> DNA
   <213> Artificial Sequence
<220>
   <221> source
   <223> /note="Description of Artificial Sequence: Synthetic oligonucleotide"
<400> 65
   aatgatacgg cgaccaccga gatctacact ggaccataca ctctttccct acacgacgct 60
   ct 62
<210> 66
   <211> 62
   <212> DNA
   <213> Artificial Sequence
<220>
   <221> source
   <223> /note="Description of Artificial Sequence: Synthetic oligonucleotide"
<400> 66
   aatgatacgg cgaccaccga gatctacacc atctgctaca ctctttccct acacgacgct 60
   ct 62
<210> 67
   <211> 62
   <212> DNA
   <213> Artificial Sequence
<220>
   <221> source
   <223> /note="Description of Artificial Sequence: Synthetic oligonucleotide"
<400> 67
   aatgatacgg cgaccaccga gatctacacg actacgaaca ctctttccct acacgacgct 60
   ct 62
<210> 68
   <211> 62
   <212> DNA
   <213> Artificial Sequence
<220>
   <221> source
   <223> /note="Description of Artificial Sequence: Synthetic oligonucleotide"
<400> 68
   aatgatacgg cgaccaccga gatctacacg cttcacaaca ctctttccct acacgacgct 60
   ct 62
<210> 69
   <211> 62
   <212> DNA
   <213> Artificial Sequence
<220>
   <221> source
   <223> /note="Description of Artificial Sequence: Synthetic oligonucleotide"
<400> 69
   aatgatacgg cgaccaccga gatctacaca acgtagcaca ctctttccct acacgacgct 60
   ct 62
<210> 70
   <211> 62
   <212> DNA
   <213> Artificial Sequence
<220>
   <221> source
   <223> /note="Description of Artificial Sequence: Synthetic oligonucleotide"
<400> 70
   aatgatacgg cgaccaccga gatctacaca ccatgtcaca ctctttccct acacgacgct      60
   ct                                                                      62
<210> 71
   <211> 62
   <212> DNA
   <213> Artificial Sequence
<220>
   <221> source
   <223> /note="Description of Artificial Sequence: Synthetic oligonucleotide"
<400> 71
   aatgatacgg cgaccaccga gatctacacc tgtggtaaca ctctttccct acacgacgct      60
   ct                                                                      62
<210> 72
   <211> 62
   <212> DNA
   <213> Artificial Sequence
<220>
   <221> source
   <223> /note="Description of Artificial Sequence: Synthetic oligonucleotide"
<400> 72
   aatgatacgg cgaccaccga gatctacacg ttggcataca ctctttccct acacgacgct      60
   ct                                                                      62
<210> 73
   <211> 62
   <212> DNA
   <213> Artificial Sequence
<220>
   <221> source
   <223> /note="Description of Artificial Sequence: Synthetic oligonucleotide"
<400> 73
   aatgatacgg cgaccaccga gatctacacg atacctgaca ctctttccct acacgacgct      60
   ct                                                                      62
<210> 74
   <211> 62
   <212> DNA
   <213> Artificial Sequence
<220>
   <221> source
   <223> /note="Description of Artificial Sequence: Synthetic oligonucleotide"
<400> 74
   aatgatacgg cgaccaccga gatctacacg acgtcataca ctctttccct acacgacgct 60
   ct 62
<210> 75
   <211> 62
   <212> DNA
   <213> Artificial Sequence
<220>
   <221> source
   <223> /note="Description of Artificial Sequence: Synthetic oligonucleotide"
<400> 75
   aatgatacgg cgaccaccga gatctacacc aggatgtaca ctctttccct acacgacgct 60
   ct 62
<210> 76
   <211> 62
   <212> DNA
   <213> Artificial Sequence
<220>
   <221> source
   <223> /note="Description of Artificial Sequence: Synthetic oligonucleotide"
<400> 76
   aatgatacgg cgaccaccga gatctacaca caccgataca ctctttccct acacgacgct 60
   ct 62
<210> 77
   <211> 62
   <212> DNA
   <213> Artificial Sequence
<220>
   <221> source
   <223> /note="Description of Artificial Sequence: Synthetic oligonucleotide"
<400> 77
   aatgatacgg cgaccaccga gatctacact gcttgctaca ctctttccct acacgacgct 60
   ct 62
<210> 78
   <211> 62
   <212> DNA
   <213> Artificial Sequence
<220>
   <221> source
   <223> /note="Description of Artificial Sequence: Synthetic oligonucleotide"
<400> 78
   aatgatacgg cgaccaccga gatctacact ggaagcaaca ctctttccct acacgacgct 60
   ct 62
<210> 79
   <211> 62
   <212> DNA
   <213> Artificial Sequence
<220>
   <221> source
   <223> /note="Description of Artificial Sequence: Synthetic oligonucleotide"
<400> 79
   aatgatacgg cgaccaccga gatctacact atgaccgaca ctctttccct acacgacgct 60
   ct 62
<210> 80
   <211> 62
   <212> DNA
   <213> Artificial Sequence
<220>
   <221> source
   <223> /note="Description of Artificial Sequence: Synthetic oligonucleotide"
<400> 80
   aatgatacgg cgaccaccga gatctacacc cgcttaaaca ctctttccct acacgacgct 60
   ct 62
<210> 81
   <211> 62
   <212> DNA
   <213> Artificial Sequence
<220>
   <221> source
   <223> /note="Description of Artificial Sequence: Synthetic oligonucleotide"
<400> 81
   aatgatacgg cgaccaccga gatctacacc ctcgttaaca ctctttccct acacgacgct 60
   ct 62
<210> 82
   <211> 62
   <212> DNA
   <213> Artificial Sequence
<220>
   <221> source
   <223> /note="Description of Artificial Sequence: Synthetic oligonucleotide"
<400> 82
   aatgatacgg cgaccaccga gatctacaca gctaagcaca ctctttccct acacgacgct 60
   ct 62
<210> 83
   <211> 62
   <212> DNA
   <213> Artificial Sequence
<220>
   <221> source
   <223> /note="Description of Artificial Sequence: Synthetic oligonucleotide"
<400> 83
   aatgatacgg cgaccaccga gatctacacc taagaccaca ctctttccct acacgacgct 60
   ct 62
<210> 84
   <211> 62
   <212> DNA
   <213> Artificial Sequence
<220>
   <221> source
   <223> /note="Description of Artificial Sequence: Synthetic oligonucleotide"
<400> 84
   aatgatacgg cgaccaccga gatctacact cacctagaca ctctttccct acacgacgct 60
   ct 62
<210> 85
   <211> 62
   <212> DNA
   <213> Artificial Sequence
<220>
   <221> source
   <223> /note="Description of Artificial Sequence: Synthetic oligonucleotide"
<400> 85
   aatgatacgg cgaccaccga gatctacacg cataacgaca ctctttccct acacgacgct       60
   ct                                                                      62
<210> 86
   <211> 62
   <212> DNA
   <213> Artificial Sequence
<220>
   <221> source
   <223> /note="Description of Artificial Sequence: Synthetic oligonucleotide"
<400> 86
   aatgatacgg cgaccaccga gatctacaca ggttcctaca ctctttccct acacgacgct       60
   ct                                                                      62
<210> 87
   <211> 62
   <212> DNA
   <213> Artificial Sequence
<220>
   <221> source
   <223> /note="Description of Artificial Sequence: Synthetic oligonucleotide"
<400> 87
   aatgatacgg cgaccaccga gatctacacc gagttagaca ctctttccct acacgacgct       60
   ct                                                                      62
<210> 88
   <211> 62
   <212> DNA
   <213> Artificial Sequence
<220>
   <221> source
   <223> /note="Description of Artificial Sequence: Synthetic oligonucleotide"
<400> 88
   aatgatacgg cgaccaccga gatctacact cttcgacaca ctctttccct acacgacgct       60
   ct                                                                      62
<210> 89
   <211> 62
   <212> DNA
   <213> Artificial Sequence
<220>
   <221> source
   <223> /note="Description of Artificial Sequence: Synthetic oligonucleotide"
<400> 89
   aatgatacgg cgaccaccga gatctacact actgctcaca ctctttccct acacgacgct 60
   ct 62
<210> 90
   <211> 62
   <212> DNA
   <213> Artificial Sequence
<220>
   <221> source
   <223> /note="Description of Artificial Sequence: Synthetic oligonucleotide"
<400> 90
   aatgatacgg cgaccaccga gatctacacc tgccataaca ctctttccct acacgacgct 60
   ct 62
<210> 91
   <211> 62
   <212> DNA
   <213> Artificial Sequence
<220>
   <221> source
   <223> /note="Description of Artificial Sequence: Synthetic oligonucleotide"
<400> 91
   aatgatacgg cgaccaccga gatctacacc caagtagaca ctctttccct acacgacgct 60
   ct 62
<210> 92
   <211> 62
   <212> DNA
   <213> Artificial Sequence
<220>
   <221> source
   <223> /note="Description of Artificial Sequence: Synthetic oligonucleotide"
<400> 92
   aatgatacgg cgaccaccga gatctacacg accgataaca ctctttccct acacgacgct 60
   ct 62
<210> 93
   <211> 62
   <212> DNA
   <213> Artificial Sequence
<220>
   <221> source
   <223> /note="Description of Artificial Sequence: Synthetic oligonucleotide"
<400> 93
   aatgatacgg cgaccaccga gatctacacc atacggaaca ctctttccct acacgacgct 60
   ct 62
<210> 94
   <211> 62
   <212> DNA
   <213> Artificial Sequence
<220>
   <221> source
   <223> /note="Description of Artificial Sequence: Synthetic oligonucleotide"
<400> 94
   aatgatacgg cgaccaccga gatctacact ctagtccaca ctctttccct acacgacgct 60
   ct 62
<210> 95
   <211> 62
   <212> DNA
   <213> Artificial Sequence
<220>
   <221> source
   <223> /note="Description of Artificial Sequence: Synthetic oligonucleotide"
<400> 95
   aatgatacgg cgaccaccga gatctacaca gtgacctaca ctctttccct acacgacgct 60
   ct 62
<210> 96
   <211> 62
   <212> DNA
   <213> Artificial Sequence
<220>
   <221> source
   <223> /note="Description of Artificial Sequence: Synthetic oligonucleotide"
<400> 96
   aatgatacgg cgaccaccga gatctacaca cctagacaca ctctttccct acacgacgct 60
   ct 62
<210> 97
   <211> 62
   <212> DNA
   <213> Artificial Sequence
<220>
   <221> source
   <223> /note="Description of Artificial Sequence: Synthetic oligonucleotide"
<400> 97
   aatgatacgg cgaccaccga gatctacacg tggtatgaca ctctttccct acacgacgct 60
   ct 62
<210> 98
   <211> 62
   <212> DNA
   <213> Artificial Sequence
<220>
   <221> source
   <223> /note="Description of Artificial Sequence: Synthetic oligonucleotide"
<400> 98
   aatgatacgg cgaccaccga gatctacacg ttatggcaca ctctttccct acacgacgct 60
   ct 62
<210> 99
   <211> 62
   <212> DNA
   <213> Artificial Sequence
<220>
   <221> source
   <223> /note="Description of Artificial Sequence: Synthetic oligonucleotide"
<400> 99
   aatgatacgg cgaccaccga gatctacaca acagcgaaca ctctttccct acacgacgct 60
   ct 62
<210> 100
   <211> 62
   <212> DNA
   <213> Artificial Sequence
<220>
   <221> source
   <223> /note="Description of Artificial Sequence: Synthetic oligonucleotide"
<400> 100
   aatgatacgg cgaccaccga gatctacacg tcctgttaca ctctttccct acacgacgct 60
   ct 62
<210> 101
   <211> 55
   <212> DNA
   <213> Artificial Sequence
<220>
   <221> source
   <223> /note="Description of Artificial Sequence: Synthetic oligonucleotide"
<400> 101
   caagcagaag acggcatacg agatgcgaat tggtgactgg agttcagacg tgtgc 55
<210> 102
   <211> 55
   <212> DNA
   <213> Artificial Sequence
<220>
   <221> source
   <223> /note="Description of Artificial Sequence: Synthetic oligonucleotide"
<400> 102
   caagcagaag acggcatacg agataaccag aggtgactgg agttcagacg tgtgc 55
<210> 103
   <211> 55
   <212> DNA
   <213> Artificial Sequence
<220>
   <221> source
   <223> /note="Description of Artificial Sequence: Synthetic oligonucleotide"
<400> 103
   caagcagaag acggcatacg agataatgcc gagtgactgg agttcagacg tgtgc 55
<210> 104
   <211> 55
   <212> DNA
   <213> Artificial Sequence
<220>
   <221> source
   <223> /note="Description of Artificial Sequence: Synthetic oligonucleotide"
<400> 104
   caagcagaag acggcatacg agatcgaagc ttgtgactgg agttcagacg tgtgc 55
<210> 105
   <211> 55
   <212> DNA
   <213> Artificial Sequence
<220>
   <221> source
   <223> /note="Description of Artificial Sequence: Synthetic oligonucleotide"
<400> 105
   caagcagaag acggcatacg agatggacag tagtgactgg agttcagacg tgtgc 55
<210> 106
   <211> 55
   <212> DNA
   <213> Artificial Sequence
<220>
   <221> source
   <223> /note="Description of Artificial Sequence: Synthetic oligonucleotide"
<400> 106
   caagcagaag acggcatacg agattacgtg ctgtgactgg agttcagacg tgtgc 55
<210> 107
   <211> 55
   <212> DNA
   <213> Artificial Sequence
<220>
   <221> source
   <223> /note="Description of Artificial Sequence: Synthetic oligonucleotide"
<400> 107
   caagcagaag acggcatacg agatactgaa gcgtgactgg agttcagacg tgtgc 55
<210> 108
   <211> 55
   <212> DNA
   <213> Artificial Sequence
<220>
   <221> source
   <223> /note="Description of Artificial Sequence: Synthetic oligonucleotide"
<400> 108
   caagcagaag acggcatacg agattgccta aggtgactgg agttcagacg tgtgc 55
<210> 109
   <211> 55
   <212> DNA
   <213> Artificial Sequence
<220>
   <221> source
   <223> /note="Description of Artificial Sequence: Synthetic oligonucleotide"
<400> 109
   caagcagaag acggcatacg agattcgtga aggtgactgg agttcagacg tgtgc 55
<210> 110
   <211> 55
   <212> DNA
   <213> Artificial Sequence
<220>
   <221> source
   <223> /note="Description of Artificial Sequence: Synthetic oligonucleotide"
<400> 110
   caagcagaag acggcatacg agatagttcc tggtgactgg agttcagacg tgtgc 55
<210> 111
   <211> 55
   <212> DNA
   <213> Artificial Sequence
<220>
   <221> source
   <223> /note="Description of Artificial Sequence: Synthetic oligonucleotide"
<400> 111
   caagcagaag acggcatacg agatgtgtgt acgtgactgg agttcagacg tgtgc 55
<210> 112
   <211> 55
   <212> DNA
   <213> Artificial Sequence
<220>
   <221> source
   <223> /note="Description of Artificial Sequence: Synthetic oligonucleotide"
<400> 112
   caagcagaag acggcatacg agatcttctc cagtgactgg agttcagacg tgtgc 55
<210> 113
   <211> 55
   <212> DNA
   <213> Artificial Sequence
<220>
   <221> source
   <223> /note="Description of Artificial Sequence: Synthetic oligonucleotide"
<400> 113
   caagcagaag acggcatacg agatactagc ctgtgactgg agttcagacg tgtgc 55
<210> 114
   <211> 55
   <212> DNA
   <213> Artificial Sequence
<220>
   <221> source
   <223> /note="Description of Artificial Sequence: Synthetic oligonucleotide"
<400> 114
   caagcagaag acggcatacg agatctctat gcgtgactgg agttcagacg tgtgc 55
<210> 115
   <211> 55
   <212> DNA
   <213> Artificial Sequence
<220>
   <221> source
   <223> /note="Description of Artificial Sequence: Synthetic oligonucleotide"
<400> 115
   caagcagaag acggcatacg agatgcaacg ttgtgactgg agttcagacg tgtgc 55
<210> 116
   <211> 55
   <212> DNA
   <213> Artificial Sequence
<220>
   <221> source
   <223> /note="Description of Artificial Sequence: Synthetic oligonucleotide"
<400> 116
   caagcagaag acggcatacg agatcggata gtgtgactgg agttcagacg tgtgc 55
<210> 117
   <211> 55
   <212> DNA
   <213> Artificial Sequence
<220>
   <221> source
   <223> /note="Description of Artificial Sequence: Synthetic oligonucleotide"
<400> 117
   caagcagaag acggcatacg agatcctgac aagtgactgg agttcagacg tgtgc 55
<210> 118
   <211> 55
   <212> DNA
   <213> Artificial Sequence
<220>
   <221> source
   <223> /note="Description of Artificial Sequence: Synthetic oligonucleotide"
<400> 118
   caagcagaag acggcatacg agatcacaag gagtgactgg agttcagacg tgtgc 55
<210> 119
   <211> 55
   <212> DNA
   <213> Artificial Sequence
<220>
   <221> source
   <223> /note="Description of Artificial Sequence: Synthetic oligonucleotide"
<400> 119
   caagcagaag acggcatacg agatgtactc ctgtgactgg agttcagacg tgtgc 55
<210> 120
   <211> 55
   <212> DNA
   <213> Artificial Sequence
<220>
   <221> source
   <223> /note="Description of Artificial Sequence: Synthetic oligonucleotide"
<400> 120
   caagcagaag acggcatacg agatagacga gagtgactgg agttcagacg tgtgc 55
<210> 121
   <211> 55
   <212> DNA
   <213> Artificial Sequence
<220>
   <221> source
   <223> /note="Description of Artificial Sequence: Synthetic oligonucleotide"
<400> 121
   caagcagaag acggcatacg agatgaagca aggtgactgg agttcagacg tgtgc 55
<210> 122
   <211> 55
   <212> DNA
   <213> Artificial Sequence
<220>
   <221> source
   <223> /note="Description of Artificial Sequence: Synthetic oligonucleotide"
<400> 122
   caagcagaag acggcatacg agatctcgag ttgtgactgg agttcagacg tgtgc 55
<210> 123
   <211> 55
   <212> DNA
   <213> Artificial Sequence
<220>
   <221> source
   <223> /note="Description of Artificial Sequence: Synthetic oligonucleotide"
<400> 123
   caagcagaag acggcatacg agatctaagt cggtgactgg agttcagacg tgtgc 55
<210> 124
   <211> 55
   <212> DNA
   <213> Artificial Sequence
<220>
   <221> source
   <223> /note="Description of Artificial Sequence: Synthetic oligonucleotide"
<400> 124
   caagcagaag acggcatacg agatgcgtta gagtgactgg agttcagacg tgtgc 55
<210> 125
   <211> 55
   <212> DNA
   <213> Artificial Sequence
<220>
   <221> source
   <223> /note="Description of Artificial Sequence: Synthetic oligonucleotide"
<400> 125
   caagcagaag acggcatacg agatggttca acgtgactgg agttcagacg tgtgc 55
<210> 126
   <211> 55
   <212> DNA
   <213> Artificial Sequence
<220>
   <221> source
   <223> /note="Description of Artificial Sequence: Synthetic oligonucleotide"
<400> 126
   caagcagaag acggcatacg agatacgtgg ttgtgactgg agttcagacg tgtgc 55
<210> 127
   <211> 55
   <212> DNA
   <213> Artificial Sequence
<220>
   <221> source
   <223> /note="Description of Artificial Sequence: Synthetic oligonucleotide"
<400> 127
   caagcagaag acggcatacg agataccatg tcgtgactgg agttcagacg tgtgc 55
<210> 128
   <211> 55
   <212> DNA
   <213> Artificial Sequence
<220>
   <221> source
   <223> /note="Description of Artificial Sequence: Synthetic oligonucleotide"
<400> 128
   caagcagaag acggcatacg agatgagtct cagtgactgg agttcagacg tgtgc 55
<210> 129
   <211> 55
   <212> DNA
   <213> Artificial Sequence
<220>
   <221> source
   <223> /note="Description of Artificial Sequence: Synthetic oligonucleotide"
<400> 129
   caagcagaag acggcatacg agattgatcc tcgtgactgg agttcagacg tgtgc 55
<210> 130
   <211> 55
   <212> DNA
   <213> Artificial Sequence
<220>
   <221> source
   <223> /note="Description of Artificial Sequence: Synthetic oligonucleotide"
<400> 130
   caagcagaag acggcatacg agatcataac cggtgactgg agttcagacg tgtgc 55
<210> 131
   <211> 55
   <212> DNA
   <213> Artificial Sequence
<220>
   <221> source
   <223> /note="Description of Artificial Sequence: Synthetic oligonucleotide"
<400> 131
   caagcagaag acggcatacg agattctact ccgtgactgg agttcagacg tgtgc 55
<210> 132
   <211> 55
   <212> DNA
   <213> Artificial Sequence
<220>
   <221> source
   <223> /note="Description of Artificial Sequence: Synthetic oligonucleotide"
<400> 132
   caagcagaag acggcatacg agattgtcga gtgtgactgg agttcagacg tgtgc 55
<210> 133
   <211> 55
   <212> DNA
   <213> Artificial Sequence
<220>
   <221> source
   <223> /note="Description of Artificial Sequence: Synthetic oligonucleotide"
<400> 133
   caagcagaag acggcatacg agatctactc tggtgactgg agttcagacg tgtgc 55
<210> 134
   <211> 55
   <212> DNA
   <213> Artificial Sequence
<220>
   <221> source
   <223> /note="Description of Artificial Sequence: Synthetic oligonucleotide"
<400> 134
   caagcagaag acggcatacg agatacgcga atgtgactgg agttcagacg tgtgc 55
<210> 135
   <211> 55
   <212> DNA
   <213> Artificial Sequence
<220>
   <221> source
   <223> /note="Description of Artificial Sequence: Synthetic oligonucleotide"
<400> 135
   caagcagaag acggcatacg agataccatc gagtgactgg agttcagacg tgtgc 55
<210> 136
   <211> 55
   <212> DNA
   <213> Artificial Sequence
<220>
   <221> source
   <223> /note="Description of Artificial Sequence: Synthetic oligonucleotide"
<400> 136
   caagcagaag acggcatacg agatgtcact tcgtgactgg agttcagacg tgtgc 55
<210> 137
   <211> 55
   <212> DNA
   <213> Artificial Sequence
<220>
   <221> source
   <223> /note="Description of Artificial Sequence: Synthetic oligonucleotide"
<400> 137
   caagcagaag acggcatacg agatcatgag aggtgactgg agttcagacg tgtgc 55
<210> 138
   <211> 55
   <212> DNA
   <213> Artificial Sequence
<220>
   <221> source
   <223> /note="Description of Artificial Sequence: Synthetic oligonucleotide"
<400> 138
   caagcagaag acggcatacg agatagattg cggtgactgg agttcagacg tgtgc 55
<210> 139
   <211> 55
   <212> DNA
   <213> Artificial Sequence
<220>
   <221> source
   <223> /note="Description of Artificial Sequence: Synthetic oligonucleotide"
<400> 139
   caagcagaag acggcatacg agatgtaacg gagtgactgg agttcagacg tgtgc 55
<210> 140
   <211> 55
   <212> DNA
   <213> Artificial Sequence
<220>
   <221> source
   <223> /note="Description of Artificial Sequence: Synthetic oligonucleotide"
<400> 140
   caagcagaag acggcatacg agatcaacat cggtgactgg agttcagacg tgtgc 55
<210> 141
   <211> 55
   <212> DNA
   <213> Artificial Sequence
<220>
   <221> source
   <223> /note="Description of Artificial Sequence: Synthetic oligonucleotide"
<400> 141
   caagcagaag acggcatacg agatggatga ctgtgactgg agttcagacg tgtgc 55
<210> 142
   <211> 55
   <212> DNA
   <213> Artificial Sequence
<220>
   <221> source
   <223> /note="Description of Artificial Sequence: Synthetic oligonucleotide"
<400> 142
   caagcagaag acggcatacg agattccgta tcgtgactgg agttcagacg tgtgc 55
<210> 143
   <211> 55
   <212> DNA
   <213> Artificial Sequence
<220>
   <221> source
   <223> /note="Description of Artificial Sequence: Synthetic oligonucleotide"
<400> 143
   caagcagaag acggcatacg agatagtgtc gtgtgactgg agttcagacg tgtgc 55
<210> 144
   <211> 55
   <212> DNA
   <213> Artificial Sequence
<220>
   <221> source
   <223> /note="Description of Artificial Sequence: Synthetic oligonucleotide"
<400> 144
   caagcagaag acggcatacg agattagaag ccgtgactgg agttcagacg tgtgc 55
<210> 145
   <211> 55
   <212> DNA
   <213> Artificial Sequence
<220>
   <221> source
   <223> /note="Description of Artificial Sequence: Synthetic oligonucleotide"
<400> 145
   caagcagaag acggcatacg agatgttcca gagtgactgg agttcagacg tgtgc 55
<210> 146
   <211> 55
   <212> DNA
   <213> Artificial Sequence
<220>
   <221> source
   <223> /note="Description of Artificial Sequence: Synthetic oligonucleotide"
<400> 146
   caagcagaag acggcatacg agatgttcgt cagtgactgg agttcagacg tgtgc 55
<210> 147
   <211> 55
   <212> DNA
   <213> Artificial Sequence
<220>
   <221> source
   <223> /note="Description of Artificial Sequence: Synthetic oligonucleotide"
<400> 147
   caagcagaag acggcatacg agatcggtga atgtgactgg agttcagacg tgtgc 55
<210> 148
   <211> 55
   <212> DNA
   <213> Artificial Sequence
<220>
   <221> source
   <223> /note="Description of Artificial Sequence: Synthetic oligonucleotide"
<400> 148
   caagcagaag acggcatacg agattgtaac gcgtgactgg agttcagacg tgtgc 55
<210> 149
   <211> 55
   <212> DNA
   <213> Artificial Sequence
<220>
   <221> source
   <223> /note="Description of Artificial Sequence: Synthetic oligonucleotide"
<400> 149
   caagcagaag acggcatacg agatttgcca gtgtgactgg agttcagacg tgtgc 55
<210> 150
   <211> 55
   <212> DNA
   <213> Artificial Sequence
<220>
   <221> source
   <223> /note="Description of Artificial Sequence: Synthetic oligonucleotide"
<400> 150
   caagcagaag acggcatacg agattaagct ccgtgactgg agttcagacg tgtgc 55
<210> 151
   <211> 55
   <212> DNA
   <213> Artificial Sequence
<220>
   <221> source
   <223> /note="Description of Artificial Sequence: Synthetic oligonucleotide"
<400> 151
   caagcagaag acggcatacg agatcatctc gtgtgactgg agttcagacg tgtgc 55
<210> 152
   <211> 55
   <212> DNA
   <213> Artificial Sequence
<220>
   <221> source
   <223> /note="Description of Artificial Sequence: Synthetic oligonucleotide"
<400> 152
   caagcagaag acggcatacg agatcattgc tggtgactgg agttcagacg tgtgc 55
<210> 153
   <211> 55
   <212> DNA
   <213> Artificial Sequence
<220>
   <221> source
   <223> /note="Description of Artificial Sequence: Synthetic oligonucleotide"
<400> 153
   caagcagaag acggcatacg agatggctga tagtgactgg agttcagacg tgtgc 55
<210> 154
   <211> 55
   <212> DNA
   <213> Artificial Sequence
<220>
   <221> source
   <223> /note="Description of Artificial Sequence: Synthetic oligonucleotide"
<400> 154
   caagcagaag acggcatacg agataggacg atgtgactgg agttcagacg tgtgc 55
<210> 155
   <211> 55
   <212> DNA
   <213> Artificial Sequence
<220>
   <221> source
   <223> /note="Description of Artificial Sequence: Synthetic oligonucleotide"
<400> 155
   caagcagaag acggcatacg agatgaaggc atgtgactgg agttcagacg tgtgc 55
<210> 156
   <211> 55
   <212> DNA
   <213> Artificial Sequence
<220>
   <221> source
   <223> /note="Description of Artificial Sequence: Synthetic oligonucleotide"
<400> 156
   caagcagaag acggcatacg agatgttgtg gagtgactgg agttcagacg tgtgc 55
<210> 157
   <211> 55
   <212> DNA
   <213> Artificial Sequence
<220>
   <221> source
   <223> /note="Description of Artificial Sequence: Synthetic oligonucleotide"
<400> 157
   caagcagaag acggcatacg agatatgtag ccgtgactgg agttcagacg tgtgc 55
<210> 158
   <211> 55
   <212> DNA
   <213> Artificial Sequence
<220>
   <221> source
   <223> /note="Description of Artificial Sequence: Synthetic oligonucleotide"
<400> 158
   caagcagaag acggcatacg agatttcagc tcgtgactgg agttcagacg tgtgc 55
<210> 159
   <211> 55
   <212> DNA
   <213> Artificial Sequence
<220>
   <221> source
   <223> /note="Description of Artificial Sequence: Synthetic oligonucleotide"
<400> 159
   caagcagaag acggcatacg agatccatga tggtgactgg agttcagacg tgtgc 55
<210> 160
   <211> 55
   <212> DNA
   <213> Artificial Sequence
<220>
   <221> source
   <223> /note="Description of Artificial Sequence: Synthetic oligonucleotide"
<400> 160
   caagcagaag acggcatacg agatgccgat tagtgactgg agttcagacg tgtgc 55
<210> 161
   <211> 55
   <212> DNA
   <213> Artificial Sequence
<220>
   <221> source
   <223> /note="Description of Artificial Sequence: Synthetic oligonucleotide"
<400> 161
   caagcagaag acggcatacg agattaccag gtgtgactgg agttcagacg tgtgc 55
<210> 162
   <211> 55
   <212> DNA
   <213> Artificial Sequence
<220>
   <221> source
   <223> /note="Description of Artificial Sequence: Synthetic oligonucleotide"
<400> 162
   caagcagaag acggcatacg agattcgtct acgtgactgg agttcagacg tgtgc 55
<210> 163
   <211> 55
   <212> DNA
   <213> Artificial Sequence
<220>
   <221> source
   <223> /note="Description of Artificial Sequence: Synthetic oligonucleotide"
<400> 163
   caagcagaag acggcatacg agatagcatc aggtgactgg agttcagacg tgtgc 55
<210> 164
   <211> 55
   <212> DNA
   <213> Artificial Sequence
<220>
   <221> source
   <223> /note="Description of Artificial Sequence: Synthetic oligonucleotide"
<400> 164
   caagcagaag acggcatacg agatacactt cggtgactgg agttcagacg tgtgc 55
<210> 165
   <211> 55
   <212> DNA
   <213> Artificial Sequence
<220>
   <221> source
   <223> /note="Description of Artificial Sequence: Synthetic oligonucleotide"
<400> 165
   caagcagaag acggcatacg agatcgatgc aagtgactgg agttcagacg tgtgc 55
<210> 166
   <211> 55
   <212> DNA
   <213> Artificial Sequence
<220>
   <221> source
   <223> /note="Description of Artificial Sequence: Synthetic oligonucleotide"
<400> 166
   caagcagaag acggcatacg agatctgtac cagtgactgg agttcagacg tgtgc 55
<210> 167
   <211> 55
   <212> DNA
   <213> Artificial Sequence
<220>
   <221> source
   <223> /note="Description of Artificial Sequence: Synthetic oligonucleotide"
<400> 167
   caagcagaag acggcatacg agatatggtg tcgtgactgg agttcagacg tgtgc 55
<210> 168
   <211> 55
   <212> DNA
   <213> Artificial Sequence
<220>
   <221> source
   <223> /note="Description of Artificial Sequence: Synthetic oligonucleotide"
<400> 168
   caagcagaag acggcatacg agattacggt tggtgactgg agttcagacg tgtgc 55
<210> 169
   <211> 55
   <212> DNA
   <213> Artificial Sequence
<220>
   <221> source
   <223> /note="Description of Artificial Sequence: Synthetic oligonucleotide"
<400> 169
   caagcagaag acggcatacg agatgtccat aggtgactgg agttcagacg tgtgc 55
<210> 170
   <211> 55
   <212> DNA
   <213> Artificial Sequence
<220>
   <221> source
   <223> /note="Description of Artificial Sequence: Synthetic oligonucleotide"
<400> 170
   caagcagaag acggcatacg agattactgc aggtgactgg agttcagacg tgtgc 55
<210> 171
   <211> 55
   <212> DNA
   <213> Artificial Sequence
<220>
   <221> source
   <223> /note="Description of Artificial Sequence: Synthetic oligonucleotide"
<400> 171
   caagcagaag acggcatacg agattgtagg acgtgactgg agttcagacg tgtgc 55
<210> 172
   <211> 55
   <212> DNA
   <213> Artificial Sequence
<220>
   <221> source
   <223> /note="Description of Artificial Sequence: Synthetic oligonucleotide"
<400> 172
   caagcagaag acggcatacg agattagcca cagtgactgg agttcagacg tgtgc 55
<210> 173
   <211> 55
   <212> DNA
   <213> Artificial Sequence
<220>
   <221> source
   <223> /note="Description of Artificial Sequence: Synthetic oligonucleotide"
<400> 173
   caagcagaag acggcatacg agattcgttc gtgtgactgg agttcagacg tgtgc 55
<210> 174
   <211> 55
   <212> DNA
   <213> Artificial Sequence
<220>
   <221> source
   <223> /note="Description of Artificial Sequence: Synthetic oligonucleotide"
<400> 174
   caagcagaag acggcatacg agatacgaag gtgtgactgg agttcagacg tgtgc 55
<210> 175
   <211> 55
   <212> DNA
   <213> Artificial Sequence
<220>
   <221> source
   <223> /note="Description of Artificial Sequence: Synthetic oligonucleotide"
<400> 175
   caagcagaag acggcatacg agatgccagt atgtgactgg agttcagacg tgtgc 55
<210> 176
   <211> 55
   <212> DNA
   <213> Artificial Sequence
<220>
   <221> source
   <223> /note="Description of Artificial Sequence: Synthetic oligonucleotide"
<400> 176
   caagcagaag acggcatacg agataattcg ccgtgactgg agttcagacg tgtgc 55
<210> 177
   <211> 55
   <212> DNA
   <213> Artificial Sequence
<220>
   <221> source
   <223> /note="Description of Artificial Sequence: Synthetic oligonucleotide"
<400> 177
   caagcagaag acggcatacg agatattgct ccgtgactgg agttcagacg tgtgc 55
<210> 178
   <211> 55
   <212> DNA
   <213> Artificial Sequence
<220>
   <221> source
   <223> /note="Description of Artificial Sequence: Synthetic oligonucleotide"
<400> 178
   caagcagaag acggcatacg agatcgaatc gagtgactgg agttcagacg tgtgc 55
<210> 179
   <211> 55
   <212> DNA
   <213> Artificial Sequence
<220>
   <221> source
   <223> /note="Description of Artificial Sequence: Synthetic oligonucleotide"
<400> 179
   caagcagaag acggcatacg agatccagaa tcgtgactgg agttcagacg tgtgc 55
<210> 180
   <211> 55
   <212> DNA
   <213> Artificial Sequence
<220>
   <221> source
   <223> /note="Description of Artificial Sequence: Synthetic oligonucleotide"
<400> 180
   caagcagaag acggcatacg agatgatcca ctgtgactgg agttcagacg tgtgc 55
<210> 181
   <211> 55
   <212> DNA
   <213> Artificial Sequence
<220>
   <221> source
   <223> /note="Description of Artificial Sequence: Synthetic oligonucleotide"
<400> 181
   caagcagaag acggcatacg agatgcaata cggtgactgg agttcagacg tgtgc 55
<210> 182
   <211> 55
   <212> DNA
   <213> Artificial Sequence
<220>
   <221> source
   <223> /note="Description of Artificial Sequence: Synthetic oligonucleotide"
<400> 182
   caagcagaag acggcatacg agattccttg gagtgactgg agttcagacg tgtgc 55
<210> 183
   <211> 55
   <212> DNA
   <213> Artificial Sequence
<220>
   <221> source
   <223> /note="Description of Artificial Sequence: Synthetic oligonucleotide"
<400> 183
   caagcagaag acggcatacg agatgattga gcgtgactgg agttcagacg tgtgc 55
<210> 184
   <211> 55
   <212> DNA
   <213> Artificial Sequence
<220>
   <221> source
   <223> /note="Description of Artificial Sequence: Synthetic oligonucleotide"
<400> 184
   caagcagaag acggcatacg agatcagctt ctgtgactgg agttcagacg tgtgc 55
<210> 185
   <211> 55
   <212> DNA
   <213> Artificial Sequence
<220>
   <221> source
   <223> /note="Description of Artificial Sequence: Synthetic oligonucleotide"
<400> 185
   caagcagaag acggcatacg agatctcgtc atgtgactgg agttcagacg tgtgc 55
<210> 186
   <211> 55
   <212> DNA
   <213> Artificial Sequence
<220>
   <221> source
   <223> /note="Description of Artificial Sequence: Synthetic oligonucleotide"
<400> 186
   caagcagaag acggcatacg agatataccg tcgtgactgg agttcagacg tgtgc 55
<210> 187
   <211> 55
   <212> DNA
   <213> Artificial Sequence
<220>
   <221> source
   <223> /note="Description of Artificial Sequence: Synthetic oligonucleotide"
<400> 187
   caagcagaag acggcatacg agatgatgaa ccgtgactgg agttcagacg tgtgc 55
<210> 188
   <211> 55
   <212> DNA
   <213> Artificial Sequence
<220>
   <221> source
   <223> /note="Description of Artificial Sequence: Synthetic oligonucleotide"
<400> 188
   caagcagaag acggcatacg agatatagcc aggtgactgg agttcagacg tgtgc 55
<210> 189
   <211> 55
   <212> DNA
   <213> Artificial Sequence
<220>
   <221> source
   <223> /note="Description of Artificial Sequence: Synthetic oligonucleotide"
<400> 189
   caagcagaag acggcatacg agataggcat acgtgactgg agttcagacg tgtgc 55
<210> 190
   <211> 55
   <212> DNA
   <213> Artificial Sequence
<220>
   <221> source
   <223> /note="Description of Artificial Sequence: Synthetic oligonucleotide"
<400> 190
   caagcagaag acggcatacg agatcctgat ctgtgactgg agttcagacg tgtgc 55
<210> 191
   <211> 55
   <212> DNA
   <213> Artificial Sequence
<220>
   <221> source
   <223> /note="Description of Artificial Sequence: Synthetic oligonucleotide"
<400> 191
   caagcagaag acggcatacg agattccagt gagtgactgg agttcagacg tgtgc 55
<210> 192
   <211> 55
   <212> DNA
   <213> Artificial Sequence
<220>
   <221> source
   <223> /note="Description of Artificial Sequence: Synthetic oligonucleotide"
<400> 192
   caagcagaag acggcatacg agatcagatc cagtgactgg agttcagacg tgtgc 55
<210> 193
   <211> 55
   <212> DNA
   <213> Artificial Sequence
<220>
   <221> source
   <223> /note="Description of Artificial Sequence: Synthetic oligonucleotide"
<400> 193
   caagcagaag acggcatacg agatgtatgg tggtgactgg agttcagacg tgtgc 55
<210> 194
   <211> 55
   <212> DNA
   <213> Artificial Sequence
<220>
   <221> source
   <223> /note="Description of Artificial Sequence: Synthetic oligonucleotide"
<400> 194
   caagcagaag acggcatacg agatcggtat tggtgactgg agttcagacg tgtgc 55
<210> 195
   <211> 55
   <212> DNA
   <213> Artificial Sequence
<220>
   <221> source
   <223> /note="Description of Artificial Sequence: Synthetic oligonucleotide"
<400> 195
   caagcagaag acggcatacg agatagcgac aagtgactgg agttcagacg tgtgc 55
<210> 196
   <211> 55
   <212> DNA
   <213> Artificial Sequence
<220>
   <221> source
   <223> /note="Description of Artificial Sequence: Synthetic oligonucleotide"
<400> 196
   caagcagaag acggcatacg agatttgtcc tggtgactgg agttcagacg tgtgc 55
<210> 197
   <211> 66
   <212> DNA
   <213> Artificial Sequence
<220>
   <221> source
   <223> /note="Description of Artificial Sequence: Synthetic primer"
<400> 197
   aatgatacgg cgaccaccga gatctacacg ttaagcgtct ttccctacac gacgctcttc 60
   cgatct 66
<210> 198
   <211> 66
   <212> DNA
   <213> Artificial Sequence
<220>
   <221> source
   <223> /note="Description of Artificial Sequence: Synthetic primer"
<400> 198
   caagcagaag acggcatacg agatgcgaat tggtgactgg agttcagacg tgtgctcttc 60
   cgatct 66
<210> 199
   <211> 43
   <212> DNA
   <213> Artificial Sequence
<220>
   <221> source
   <223> /note="Description of Artificial Sequence: Synthetic oligonucleotide"
<400> 199
   tctttcccta cacgacgctc ttccgatctc agatctgatt aca 43
<210> 200
   <211> 48
   <212> DNA
   <213> Artificial Sequence
<220>
   <221> source
   <223> /note="Description of Artificial Sequence: Synthetic oligonucleotide"
<400> 200
   gattacactt ttgacagatc ggaagagaca cgtctgaact ccagtcac 48
<210> 201
   <211> 48
   <212> DNA
   <213> Artificial Sequence
<220>
   <221> source
   <223> /note="Description of Artificial Sequence: Synthetic oligonucleotide"
<400> 201
   tgtaatcaga tctgagatcg gaagagcaca cgtctgaact ccagtcac 48
<210> 202
   <211> 44
   <212> DNA
   <213> Artificial Sequence
<220>
   <221> source
   <223> /note="Description of Artificial Sequence: Synthetic oligonucleotide"
<400> 202
   tctttcccta cacgacgctc ttccgatctg tcaaaagtgt aatc 44
<210> 203
   <211> 49
   <212> DNA
   <213> Artificial Sequence
<220>
   <221> source
   <223> /note="Description of Artificial Sequence: Synthetic oligonucleotide"
<400> 203
   gattacactt ttgacagatc ggaagagcac acgtctgaac tccagtcac 49
<210> 204
   <211> 43
   <212> DNA
   <213> Artificial Sequence
<220>
   <221> source
   <223> /note="Description of Artificial Sequence: Synthetic oligonucleotide"
<400> 204
   tgtaatcaga tctgagatcg gaagagcgtc gtgtagggaa aga 43
<210> 205
   <211> 44
   <212> DNA
   <213> Artificial Sequence
<220>
   <221> source
   <223> /note="Description of Artificial Sequence: Synthetic oligonucleotide"
<400> 205
   gattacactt ttgacagatc ggaagagcgt cgtgtaggga aaga 44
<210> 206
   <211> 66
   <212> DNA
   <213> Artificial Sequence
<220>
   <221> source
   <223> /note="Description of Artificial Sequence: Synthetic oligonucleotide"
<400> 206
   aatgatacgg cgaccaccga gatctacacc ttaagcgtct ttccctacac gacgctcttc 60
   cgatct 66
<210> 207
   <211> 80
   <212> DNA
   <213> Artificial Sequence
<220>
   <221> source
   <223> /note="Description of Artificial Sequence: Synthetic oligonucleotide"
<400> 207
   aatgatacgg cgaccaccga gatctacacg ttaagcgtct ttccctacac gacgctcttc 60
   cgatctcaga tctgattaca 80
<210> 208
   <211> 80
   <212> DNA
   <213> Artificial Sequence
<220>
   <221> source
   <223> /note="Description of Artificial Sequence: Synthetic oligonucleotide"
<400> 208
   gattacactt ttacagatcg gaagagcaca cgtctgaact ccagtcacca attcgcatct 60
   cgtatgccgt cttctgcttg 80
<210> 209
   <211> 80
   <212> DNA
   <213> Artificial Sequence
<220>
   <221> source
   <223> /note="Description of Artificial Sequence: Synthetic oligonucleotide"
<400> 209
   gttcgtcttc tgccgtatgc tctacgctta accactgacc tcaagtctgc acacgacaag 60
   gctagagtct agactaatgt 80
<210> 210
   <211> 81
   <212> DNA
   <213> Artificial Sequence
<220>
   <221> source
   <223> /note="Description of Artificial Sequence: Synthetic oligonucleotide"
<400> 210
   ctaatgtgaa aactgtctag ccttctcgca gcacatccct ttctgcgaat tccacatcta 60
   gagccaccag cggcatagta a 81
<210> 211
   <211> 81
   <212> DNA
   <213> Artificial Sequence
<220>
   <221> source
   <223> /note="Description of Artificial Sequence: Synthetic oligonucleotide"
<400> 211
   gattacactt ttgacagatc ggaagagcac acgtctgaac tccagtcacc aattcgcatc 60
   tcgtatgccg tcttctgctt g 81
<210> 212
   <211> 80
   <212> DNA
   <213> Artificial Sequence
<220>
   <221> source
   <223> /note="Description of Artificial Sequence: Synthetic oligonucleotide"
<400> 212
   gttcgtcttc tgccgtatgc tctacgctta accactgacc tcaagtctgc acacgacaag 60
   gctagagtct agactaatgt 80
<210> 213
   <211> 80
   <212> DNA
   <213> Artificial Sequence
<220>
   <221> source
   <223> /note="Description of Artificial Sequence: Synthetic oligonucleotide"
<400> 213
   ttactatgcc gctggtggct ctagatgtgc aattcgcaga aagggatgtg ctgcgagaag 60
   gctagagtct agactaatgt 80
<210> 214
   <211> 80
   <212> DNA
   <213> Artificial Sequence
<220>
   <221> source
   <223> /note="Description of Artificial Sequence: Synthetic oligonucleotide"
<400> 214
   ttactatgcc gctggtggct ctagatgtgc aattcgcaga aagggatgtg ctgcgagaag 60
   gctagagtct agactaatgt 80
<210> 215
   <211> 81
   <212> DNA
   <213> Artificial Sequence
<220>
   <221> source
   <223> /note="Description of Artificial Sequence: Synthetic oligonucleotide"
<400> 215
   gattacactt ttgacagatc ggaagagcac acgtctgaac tccagtcacc aattcgcatc 60
   tcgtatgccg tcttctgctt g 81
<210> 216
   <211> 81
   <212> DNA
   <213> Artificial Sequence
<220>
   <221> source
   <223> /note="Description of Artificial Sequence: Synthetic oligonucleotide"
<400> 216
   gattacactt ttgacagatc ggaagagcgt cgtgtaggga aagacgctta aggtgtagat 60
   ctcggtggtc gccgtatcat t 81
<210> 217
   <211> 80
   <212> DNA
   <213> Artificial Sequence
<220>
   <221> source
   <223> /note="Description of Artificial Sequence: Synthetic oligonucleotide"
<400> 217
   gttcgtcttc tgccgtatgc tctacgctta accactgacc tcaagtctgc acacgacaag        60
   gctagagtct agactaatgt                                                    80

## Description

### BACKGROUND

The disclosure relates, in general, to the field of sample preparation for next generation sequencing of nucleic acids and, more particularly, to a system and method for the isolation and qualification of nucleic acids.

Forked nucleic acid adapters (also known as Y-adapters) for use with next generation sequencing (NGS) platforms (e.g., ILLUMINA sequencing-by-synthesis platforms) can include features such sample identifiers (SID) and unique identifiers (UID) that enable sample multiplexing, molecular counting, and the like. Accordingly, forked adapters can enable efficient NGS library preparation via adapter ligation methods, maximizing the number of molecules that can be sequenced in a paired-end fashion, while allowing correct counting of molecules and error reduction with UIDs. However, there are a number of challenges that may arise when producing and using adapter such as these.

In one aspect, the cost of oligo manufacturing is high. For an adapter design with 16 unique UIDs, in order to create adapters with 16 different single-stranded SIDs, 274 different oligo sequences must be produced. However, only a small number of oligo manufacturers are able to produce such a large number of different oligos at a high enough purity in a large enough scale to satisfy these specifications.

In another aspect, addition of PhiX to the final sequencing libraries (which can comprise 10-15% of the final concentration of the input molecules in an NGS experiment) effectively reduces the number of sequencing reads available for DNA molecules from the sample. PhiX DNA is often used as a spike-in control during library preparation as a quality control for NGS experiments or to add complexity in the case of less complex DNA samples. For example, PhiX may be used if 100% of the bases at positions 3 and 4 in the library sequences are G and T as PhiX increases the complexity at these positions, allowing the ILLUMINA sequencer to properly differentiate clusters and phase the molecules.

In yet another aspect, with 16 2-base UIDs (i.e., UIDs having a length of 2 nucleotides), any error in the UID results in a different acceptable UID. This could lead to over counting of molecules, and less efficient error reduction than UIDs that can be better differentiated.

In a further aspect, a known phenomenon that is often observed in NGS experiments involves the SID for a molecule from one sample attaching or otherwise associating with molecule from another sample. This can result in molecules being assigned to the incorrect sample. If the adapter scheme only contains an SID on one side of the adapter, and the SID is not directly attached to the molecule of interest, this crossover effect can perturb variant calling, thereby resulting in incorrect variant calls. Taken together with the other aforementioned challenges, it is clear that there is room for improvement of nucleic acid adapters for NGS experiments.

Accordingly, there is a need for new designs for nucleic acid adapters that enable lower manufacturing costs and greater efficiency and accuracy in NGS experiments.
WO2008/093098 discloses methods and kits for sequencing nucleic acids with the use of forked adaptors that are ligated to each end of the target nucleic acid, generating thus adapter-target-adapter constructs which are then used for amplification with primers that comprise a sample specific tag sequence.
Aaron M Newman et al: "Integrated digital error suppression for improved detection of circulating tumor DNA", NATURE BIOTECHNOLOGY, vol. 34, no. 5, 28 March 2016, pages 547-555 disclose the use of tagging DNA molecules for sequencing with unique identifiers (UID).
Brant C. Faircloth et al: "Not All Sequence Tags Are Created Equal: Designing and Validating Sequence Identification Tags Robust to Indels", PLOS ONE, vol. 7, no. 8, 10 August 2012, page e42543 describe ideal sequences for adapter molecules and refer to properties of the adapters like pairwise edit distance.

### SUMMARY

The present invention overcomes the aforementioned drawbacks by providing a kit for preparing a library of nucleic acids having adapter sequences for sequencing, the kit comprising:
a first oligonucleotide having a first tail sequence, a first common sequence, a first unique identifier sequence, and a first variable length punctuation mark;
a second oligonucleotide having a second tail sequence, a second common sequence complimentary to the first common sequence, a second unique identifier sequence complimentary to the first unique identifier sequence , and a second variable length punctuation mark complimentary to the first variable length punctuation mark;
a first primer having a first sample identifier sequence and a first priming sequence at a 3' end of the first primer, the first priming sequence including the first tail sequence of the first oligonucleotide; and
a second primer having a second sample identifier sequence and a second priming sequence at a 3' end of the second primer, the second priming sequence being complimentary to the second tail sequence of the second oligonucleotide.

The first sample identifier sequence and the second sample identifier sequence may have a one-to-one mapping.

The first variable length punctuation mark may have a length of 2-4 nucleotides.

The first variable length punctuation mark may include at least one of a G and a C nucleotide.

The first unique identifier sequence has a length of at least 5 nucleotides.
The first unique identifier sequence may have a pairwise edit distance of at least 3.

The present invention also provides kit for preparing a library of nucleic acids having adapter sequences for sequencing, the kit comprising:
a plurality of oligonucleotide pairs, each of the oligonucleotide pairs including:
   a first oligonucleotide having a first tail sequence, a first common sequence, a first unique identifier sequence, and ii) a first variable length punctuation mark, and
   a second oligonucleotide having a second tail sequence, a second common sequence complimentary to the first common sequence, a second unique identifier sequence complimentary to the first unique identifier sequence , and a second variable length punctuation mark complimentary to the first variable length punctuation mark,
   a first primer having a first sample identifier sequence and a first priming sequence at a 3' end of the first primer, the first priming sequence including the first tail sequence of the first oligonucleotide; and
   a second primer having a second sample identifier sequence and a second priming sequence at a 3' end of the second primer, the second priming sequence being complimentary to the second tail sequence of the second oligonucleotide.

Each of the first unique identifier sequences of each of the plurality of oligonucleotide pairs may be different.

Each of the first tail sequences of each of the plurality of oligonucleotide pairs may be the same.

Each of the second tail sequences of each of the plurality of oligonucleotide pairs may be the same.

The plurality of oligonucleotide pairs may be annealed to form a forked adapter.

The first sample identifier sequence and the second sample identifier sequence may have a one-to-one mapping.

The first variable length punctuation marks may have a length of 2-4 nucleotides.

Each of the first variable length punctuation marks may include at least one of a G and a C nucleotide.

Each of the first unique identifier sequences may have a length of at least 5 nucleotides.
Then, ach of the first unique identifier sequences has a pairwise edit distance of at least 3.

The present invention also provides a method of preparing a library of nucleic acid molecules, the method comprising:
attaching one of a plurality of oligonucleotide adapters to each end of a target nucleic acid to provide an adapter-target-adapter construct, each of the plurality of oligonucleotide adapters having:
   a first oligonucleotide having a first tail sequence, a first common sequence, a first unique identifier sequence, and a first variable length punctuation mark, and
   a second oligonucleotide having a second tail sequence, a second common sequence complimentary to the first common sequence, a second unique identifier sequence complimentary to the first unique identifier sequence , and a second variable length punctuation mark complimentary to the first variable length punctuation mark;
annealing a first primer to the adapter-target-adapter construct, the first primer having a first sample identifier sequence and a first priming sequence at a 3' end of the first primer, the first priming sequence including the first tail sequence of the first oligonucleotide; and
extending each of the first primer and the second primer to form extension products complementary to each strand of the adapter-target-adapter constructs.

Each of the first unique identifier sequences of each of the plurality of oligonucleotide adapters may be different.

Each of the first tail sequences of each of the plurality of oligonucleotide adapters may be the same.

Each of the second tail sequences of each of the plurality of oligonucleotide adapters may be the same.

The first sample identifier sequence and the second sample identifier sequence may have a one-to-one mapping. Then, each of the first variable length punctuation marks may have a length of 2-4 nucleotides and/or each of the first variable length punctuation marks may include at least one of a G and a C nucleotide.

Each of the first unique identifier sequences has a length of at least 5 nucleotides. Then, each of the first unique identifier sequences may have a pairwise edit distance of at least 3.

### BRIEF DESCRIPTION OF THE DRAWINGS

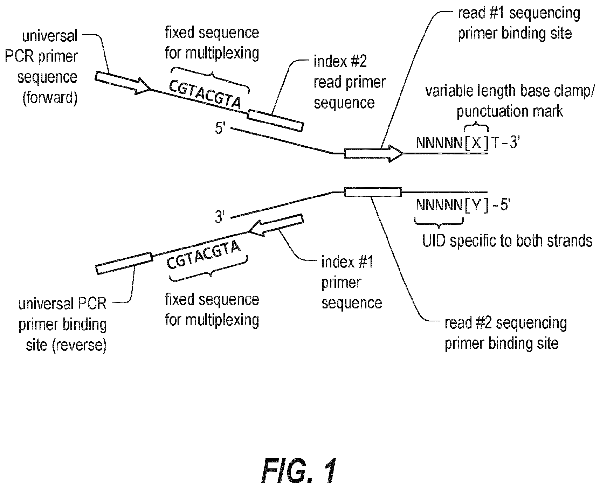

Figure 1 is a schematic diagram depicting an embodiment of the components of a modular nucleic acid adapter according to the present disclosure.

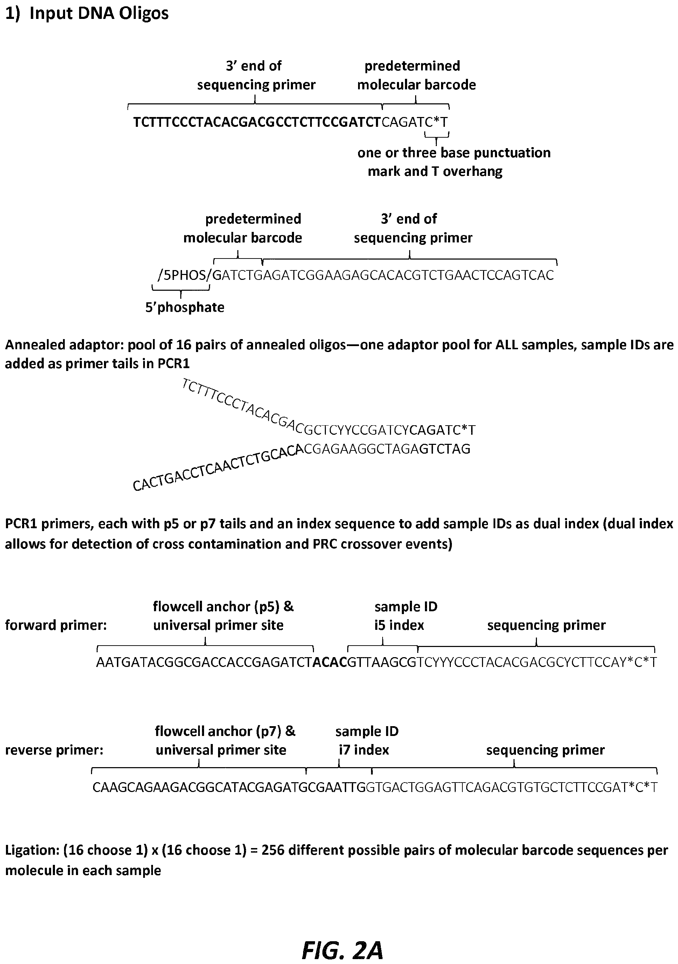

Figure 2A is a schematic illustration of a method for preparing a library of nucleic acids with the modular nucleic acid adapters according the present disclosure. In a first portion of the method, a scheme is illustrated for assembling a pool of adapter oligos, including the design of adapter oligos having predetermined molecular barcodes (UIDs) and forward and reverse primers having SIDs for amplification of the adaptor oligos following ligation to sample nucleic acid library fragments. In the present example, each sample nucleic acid fragment is ligated at each end to one of the 16 different annealed adapters (each of the annealed adapters having one of 16 predetermined molecular barcodes or UIDs). Following ligation, each nucleic acid fragment in the sample is associated with one of 256 different possible pairs of molecular barcode sequences. Fig. 2A discloses SEQ ID NOS 3,4, 3,4, 197 and 198, respectively, in the order of their appearance.

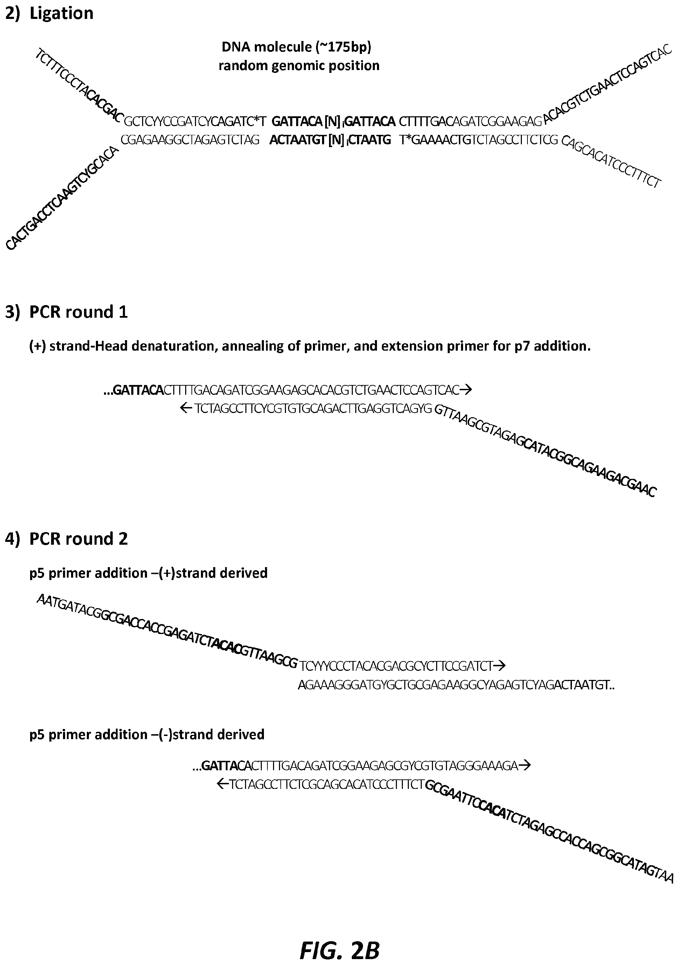

Figure 2B is a continuation of the schematic illustration of the method of FIG. 2A. Following ligation of the annealed adapters to the target DNA molecules in the nucleic acid sample, the primers having SIDs illustrated in FIG. 2A are used in first and second rounds of a polymerase chain reaction (PCR) experiment to incorporate SIDs and NGS platform specific sequences (e.g., p5 and p7 sequences for ILLUMINA sequencers). Fig. 2B discloses SEQ ID NOS 199-203, 198, 197 and 204-206, respectively, in the order of their appearance.

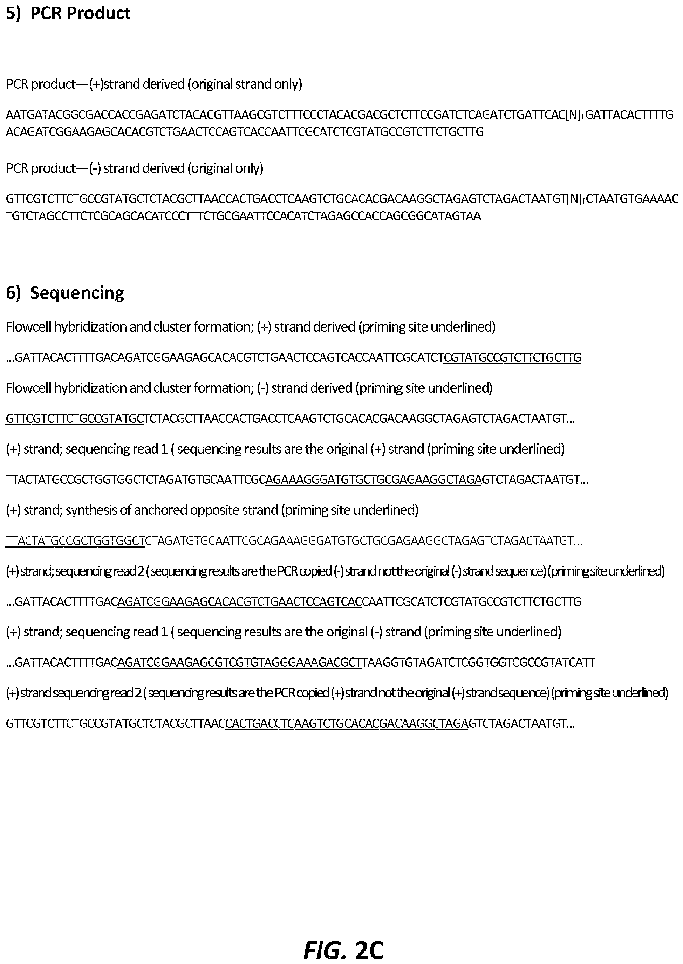

Figure 2C is a continuation of the schematic illustration of the method of FIGS. 2A and 2B. Following PCR amplification, the illustrated PCR products are subjected to sequencing. In the present example, the relevant priming sites for sequencing on an ILLUMINA platform (e.g., ILLUMINA HISEQ series) are indicated with underlining for each of the PCR products. Fig. 2C discloses SEQ ID NOS 207-217, respectively, in the order of their appearance.

### DETAILED DESCRIPTION

### I. Definitions

In this application, unless otherwise clear from context, (i) the term "a" may be understood to mean "at least one"; (ii) the term "or" may be understood to mean "and/or"; (iii) the terms "comprising" and "including" may be understood to encompass itemized components or steps whether presented by themselves or together with one or more additional components or steps; and (iv) the terms "about" and "approximately" may be understood to permit standard variation as would be understood by those of ordinary skill in the art; and (v) where ranges are provided, endpoints are included.

Approximately: As used herein, the term "approximately" or "about", as applied to one or more values of interest, refers to a value that is similar to a stated reference value. In certain embodiments, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

Associated with: Two events or entities are "associated" with one another, as that term is used herein, if the presence, level, and/or form of one is correlated with that of the other. For example, a particular entity (*e.g.,* polypeptide, genetic signature, metabolite, etc.) is considered to be associated with a particular disease, disorder, or condition, if its presence, level and/or form correlates with incidence of and/or susceptibility to the disease, disorder, or condition (*e.g*., across a relevant population). In some embodiments, two or more entities are physically "associated" with one another if they interact, directly or indirectly, so that they are and/or remain in physical proximity with one another. In some embodiments, two or more entities that are physically associated with one another are covalently linked to one another; in some embodiments, two or more entities that are physically associated with one another are not covalently linked to one another but are non-covalently associated, for example by means of hydrogen bonds, van der Waals interaction, hydrophobic interactions, magnetism, and combinations thereof.

Biological Sample: As used herein, the term "biological sample" typically refers to a sample obtained or derived from a biological source (*e.g.,* a tissue or organism or cell culture) of interest, as described herein. In some embodiments, a source of interest comprises or consists of an organism, such as an animal or human. In some embodiments, a biological sample is comprises or consists of biological tissue or fluid. In some embodiments, a biological sample may be or comprise bone marrow; blood; blood cells; ascites; tissue or fine needle biopsy samples; cell-containing body fluids; free floating nucleic acids; sputum; saliva; urine; cerebrospinal fluid, peritoneal fluid; pleural fluid; feces; lymph; gynecological fluids; skin swabs; vaginal swabs; oral swabs; nasal swabs; washings or lavages such as a ductal lavages or broncheoalveolar lavages; aspirates; scrapings; bone marrow specimens; tissue biopsy specimens; surgical specimens; other body fluids, secretions, and/or excretions; and/or cells therefrom, etc. In some embodiments, a biological sample is comprises or consists of cells obtained from an individual. In some embodiments, obtained cells are or include cells from an individual from whom the sample is obtained. In some embodiments, a sample is a "primary sample" obtained directly from a source of interest by any appropriate means. For example, in some embodiments, a primary biological sample is obtained by methods selected from the group consisting of biopsy (*e.g.,* fine needle aspiration or tissue biopsy), surgery, collection of body fluid (*e.g.,* blood, lymph, feces etc.), etc. In some embodiments, as will be clear from context, the term "sample" refers to a preparation that is obtained by processing (*e.g*., by removing one or more components of and/or by adding one or more agents to) a primary sample. For example, filtering using a semi-permeable membrane. Such a "processed sample" may comprise, for example nucleic acids or proteins extracted from a sample or obtained by subjecting a primary sample to techniques such as amplification or reverse transcription of mRNA, isolation and/or purification of certain components, etc.

Comprising: A composition or method described herein as "comprising" one or more named elements or steps is open-ended, meaning that the named elements or steps are essential, but other elements or steps may be added within the scope of the composition or method. It is to be understood that composition or method described as "comprising" (or which "comprises") one or more named elements or steps also describes the corresponding, more limited composition or method "consisting essentially of" (or which "consists essentially of") the same named elements or steps, meaning that the composition or method includes the named essential elements or steps and may also include additional elements or steps that do not materially affect the basic and novel characteristic(s) of the composition or method. It is also understood that any composition or method described herein as "comprising" or "consisting essentially of" one or more named elements or steps also describes the corresponding, more limited, and closed-ended composition or method "consisting of" (or "consists of") the named elements or steps to the exclusion of any other unnamed element or step. In any composition or method disclosed herein, known or disclosed equivalents of any named essential element or step may be substituted for that element or step.

Designed: As used herein, the term "designed" refers to an agent (i) whose structure is or was selected by the hand of man; (ii) that is produced by a process requiring the hand of man; and/or (iii) that is distinct from natural substances and other known agents.

Determine: Those of ordinary skill in the art, reading the present specification, will appreciate that "determining" can utilize or be accomplished through use of any of a variety of techniques available to those skilled in the art, including for example specific techniques explicitly referred to herein. In some embodiments, determining involves manipulation of a physical sample. In some embodiments, determining involves consideration and/or manipulation of data or information, for example utilizing a computer or other processing unit adapted to perform a relevant analysis. In some embodiments, determining involves receiving relevant information and/or materials from a source. In some embodiments, determining involves comparing one or more features of a sample or entity to a comparable reference.

Identity: As used herein, the term "identity" refers to the overall relatedness between polymeric molecules, *e.g.,* between nucleic acid molecules (*e.g.,* DNA molecules and/or RNA molecules) and/or between polypeptide molecules. In some embodiments, polymeric molecules are considered to be "substantially identical" to one another if their sequences are at least 25%, 30%, 35%, 40%, 45%, 50%, 55%, 60%, 65%, 70%, 75%, 80%, 85%, 90%, 95%, or 99% identical. Calculation of the percent identity of two nucleic acid or polypeptide sequences, for example, can be performed by aligning the two sequences for optimal comparison purposes (*e.g.,* gaps can be introduced in one or both of a first and a second sequences for optimal alignment and non-identical sequences can be disregarded for comparison purposes). In certain embodiments, the length of a sequence aligned for comparison purposes is at least 30%, at least 40%, at least 50%, at least 60%, at least 70%, at least 80%, at least 90%, at least 95%, or substantially 100% of the length of a reference sequence. The nucleotides at corresponding positions are then compared. When a position in the first sequence is occupied by the same residue (*e.g.,* nucleotide or amino acid) as the corresponding position in the second sequence, then the molecules are identical at that position. The percent identity between the two sequences is a function of the number of identical positions shared by the sequences, taking into account the number of gaps, and the length of each gap, which needs to be introduced for optimal alignment of the two sequences. The comparison of sequences and determination of percent identity between two sequences can be accomplished using a mathematical algorithm. For example, the percent identity between two nucleotide sequences can be determined using the algorithm of Meyers and Miller (CABIOS, 1989, 4: 11-17), which has been incorporated into the ALIGN program (version 2.0). In some exemplary embodiments, nucleic acid sequence comparisons made with the ALIGN program use a PAM120 weight residue table, a gap length penalty of 12 and a gap penalty of 4. The percent identity between two nucleotide sequences can, alternatively, be determined using the GAP program in the GCG software package using an NWSgapdna.CMP matrix.

Sample: As used herein, the term "sample" refers to a substance that is or contains a composition of interest for qualitative and or quantitative assessment. In some embodiments, a sample is a biological sample (*i.e.,* comes from a living thing (*e.g.,* cell or organism). In some embodiments, a sample is from a geological, aquatic, astronomical, or agricultural source. In some embodiments, a source of interest comprises or consists of an organism, such as an animal or human. In some embodiments, a sample for forensic analysis is or comprises biological tissue, biological fluid, organic or non-organic matter such as, *e.g.,* clothing, dirt, plastic, water. In some embodiments, an agricultural sample, comprises or consists of organic matter such as leaves, petals, bark, wood, seeds, plants, fruit, etc.

Substantially: As used herein, the term "substantially" refers to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "substantially" is therefore used herein to capture the potential lack of completeness inherent in many biological and chemical phenomena.

Synthetic: As used herein, the word "synthetic" means produced by the hand of man, and therefore in a form that does not exist in nature, either because it has a structure that does not exist in nature, or because it is either associated with one or more other components, with which it is not associated in nature, or not associated with one or more other components with which it is associated in nature.

Variant: As used herein, the term "variant" refers to an entity that shows significant structural identity with a reference entity but differs structurally from the reference entity in the presence or level of one or more chemical moieties as compared with the reference entity. In many embodiments, a variant also differs functionally from its reference entity. In general, whether a particular entity is properly considered to be a "variant" of a reference entity is based on its degree of structural identity with the reference entity. As will be appreciated by those skilled in the art, any biological or chemical reference entity has certain characteristic structural elements. A variant, by definition, is a distinct chemical entity that shares one or more such characteristic structural elements. To give but a few examples, a small molecule may have a characteristic core structural element (*e.g.,* a macrocycle core) and/or one or more characteristic pendent moieties so that a variant of the small molecule is one that shares the core structural element and the characteristic pendent moieties but differs in other pendent moieties and/or in types of bonds present (single vs double, E vs Z, etc.) within the core, a polypeptide may have a characteristic sequence element comprised of a plurality of amino acids having designated positions relative to one another in linear or three-dimensional space and/or contributing to a particular biological function, a nucleic acid may have a characteristic sequence element comprised of a plurality of nucleotide residues having designated positions relative to another in linear or three-dimensional space. For example, a variant polypeptide may differ from a reference polypeptide as a result of one or more differences in amino acid sequence and/or one or more differences in chemical moieties (*e.g.,* carbohydrates, lipids, etc.) covalently attached to the polypeptide backbone. In some embodiments, a variant polypeptide shows an overall sequence identity with a reference polypeptide that is at least 85%, 86%, 87%, 88%, 89%, 90%, 91%, 92%, 93%, 94%, 95%, 96%, 97%, or 99%. Alternatively or additionally, in some embodiments, a variant polypeptide does not share at least one characteristic sequence element with a reference polypeptide. In some embodiments, the reference polypeptide has one or more biological activities. In some embodiments, a variant polypeptide shares one or more of the biological activities of the reference polypeptide. In some embodiments, a variant polypeptide lacks one or more of the biological activities of the reference polypeptide. In some embodiments, a variant polypeptide shows a reduced level of one or more biological activities as compared with the reference polypeptide. In many embodiments, a polypeptide of interest is considered to be a "variant" of a parent or reference polypeptide if the polypeptide of interest has an amino acid sequence that is identical to that of the parent but for a small number of sequence alterations at particular positions. Typically, fewer than 20%, 15%, 10%, 9%, 8%, 7%, 6%, 5%, 4%, 3%, 2% of the residues in the variant are substituted as compared with the parent. In some embodiments, a variant has 10, 9, 8, 7, 6, 5, 4, 3, 2, or 1 substituted residue as compared with a parent. Often, a variant has a very small number (*e.g.,* fewer than 5, 4, 3, 2, or 1) number of substituted functional residues (*i.e.,* residues that participate in a particular biological activity). Furthermore, a variant typically has not more than 5, 4, 3, 2, or 1 additions or deletions, and often has no additions or deletions, as compared with the parent. Moreover, any additions or deletions are typically fewer than about 25, about 20, about 19, about 18, about 17, about 16, about 15, about 14, about 13, about 10, about 9, about 8, about 7, about 6, and commonly are fewer than about 5, about 4, about 3, or about 2 residues. In some embodiments, a variant may also have one or more functional defects and/or may otherwise be considered a "mutant". In some embodiments, the parent or reference polypeptide is one found in nature. As will be understood by those of ordinary skill in the art, a plurality of variants of a particular polypeptide of interest may commonly be found in nature, particularly when the polypeptide of interest is an infectious agent polypeptide.

### II. Detailed Description of Certain Embodiments

As also discussed above, in various situations it may be useful to provide adapters for nucleic acid library preparation for NGS or the like. However, current adapter designs have several drawbacks with respect to cost of manufacture, efficiency of sequencing and accuracy of downstream base-calling, sample identification, and the like.

These and other challenges may be overcome with a modular nucleic acid adapter according to the present disclosure. In one aspect, the disclosed adapters may be implemented to overcome the aforementioned challenges using a scheme whereby UIDs and SIDs are distributed onto two separate sets of oligos (FIG. 1). Accordingly, in one embodiment, a pool of forked adapter is prepared with each adapter having a UID selected from a set of two or more different UID sequences. Following ligation of the UID-containing forked adapters to target nucleic acids, the resulting ligation products are amplified with primers including SIDs, and optionally other sequence information such as NGS platform specific sequences. The resulting amplification products include both a pair of UIDs from the initial adapter ligation step and an SID (or pair of SIDs) from the amplification step. Notably, variations of the aforementioned modular design are also within the scope of the present disclosure. For example, the location of the UIDs and SIDs can be swapped. That is, the UIDs on the forked adapters can be substituted for SID and the SIDs included in the amplification primers can be substituted for UIDs. As a result, the SIDs are incorporated by ligation and the UIDs are incorporated through PCR amplification. Yet other variations of the disclosed modular nucleic acid adapters will become apparent from the following disclosure.

One advantage of the disclosed modular nucleic acid adapter design is that instead of each adapter having its own SID, then being amplified by a universal PCR primer pair, the adapter is universal (e.g., adapters with 16 different UIDs are pooled into one adapter tube), and the PCR primers contain the SIDs. In this design, the UIDs and SIDs are decoupled, allowing a reduction in the number of necessary oligos to be produced. For an adapter design with 16 different UIDs and 16 SIDs, 64 different oligos are necessary, instead of 274. In addition, these oligos are shorter than those in the previous design, which also reduces oligo synthesis costs, and may increase efficiency of ligation (and therefore assay efficiency) as well. In one aspect, the set of different UIDs includes 2, 4, 8, 16, 32, 64, 128, or more different UID sequences. In another aspect, the set of different SIDs includes 2, 4, 8, 16, 32, 64, 128, or more different SID sequences. Notably, the number of UIDs and SIDs selected will depend on the nature of the experiment including the desired number of samples for multiplexing, the capacity of the NGS platform (i.e., the sequencing instrument), the complexity of the nucleic acid sample to be analyzed, and the like.

In another aspect of the disclosed modular nucleic acid adapter design, instead of having a consistent 2-base punctuation mark of GT at the end of every adapter, the punctuation mark is synthesized with a variable length. The use of a variable length punctuation mark (FIG. 1) ensure adequate complexity at each position within the read, so a PhiX spike-in or other like control or complexity enhancing material is not needed. In the one embodiment, the punctuation mark is varied between 2- and 4- bases. In this implementation, the last base before the T-overhang is selected from a C nucleotide or a G nucleotide, thereby allowing a stronger hydrogen bond (i.e., a "G-C clamp"), which may show improved ligation efficiency. In another embodiment, the terminal base of the punctuation mark is selected from any of any nucleotide. In one aspect, the punctuation marks can be designed such that no position in the sequencing read ever has greater than a selected percentage (e.g., 62.5%) of any base at the position, removing the need for addition of PhiX or another like agent when using the disclosed adapters. A list of punctuation marks and the breakdown of base % at each position is shown in Tables 1 and 2.

**Table 1**

| **i5 punctuation marks (with T overhang)** |
|---|
| C |
| G |
| AAG |
| TCC |
| C |
| G |
| AGG |
| TAC |
| C |
| G |
| TCG |
| AGC |
| C |
| G |
| TAG |
| ACC |

**Table 2**

| **% Each base by position in the punctuation mark*** | | |
|---|---|---|
| **Base** | **Position 1** | **Position 2** |
| A | 25% | 18.75% |
| C | 25% | 18.75% |
| G | 25% | 12.50% |
| T | 25% | 50% |

| | | |
|---|---|---|
| *Assuming a nucleic acid sample having 25% representation of each base at each position | | |

In another aspect of the present disclosure, UIDs can be designed such that, if one or multiple errors occur in the UID, the UID does not result in the same sequence as another UID in the selected pool of UID sequences. In this way, UIDs with one or multiple errors can be corrected or removed from further analysis. In the attached implementation, instead of UIDs with a length of 2 nucleotides, a UID with a length of 5 nucleotides with a pairwise edit distance of at least 3 are used. As defined herein, pairwise edit distance is a measure of the similarity between two strings of characters (e.g., nucleotide sequences) as determined by counting the minimum number of operations required to transform one string into the other. As used in the examples of the present disclosure, pairwise edit distance is determined according the Levenshtein distance, in which operations are limited to deletions, insertions, and substitutions; however, pairwise edit distance may be calculated using other approaches as will be appreciated by one of ordinary skill in the art. With a pairwise edit distance of 3, UIDs having a single error can always be identified correctly. This allows for up to 25 different UIDs (see, e.g., Faircloth, et al. 2012. PLoS ONE 7(8): e42543). In the attached implementation (Table 3), 16 UIDs are used. Different length UIDs can also be used (e.g., designs with UIDs as short as 2 and as long as 10 bases in length). With 2 base UIDs and the use of a variable punctuation mark as described herein, UIDs + punctuation marks with a pairwise hamming distance of 2 can be generated-in this implementation (Table 4), one substitution error in the UID will never result in a UID + punctuation mark sequence that is identical to another UID + punctuation mark in the set. As defined herein, hamming distance is the edit distance between two strings where the only allowed operation is a substitution. Two additional UID schemes are shown in Tables 5 and 6 below.

**Table 3 (scheme 1)**

| **UID** | **rc UID** | **i5 punc** | **i7 punc** |
|---|---|---|---|
| CAGAT | ATCTG | C | G |
| GCTGA | TCAGC | G | C |
| GTCAA | TTGAC | AAG | CTT |
| GACGT | ACGTC | TCC | GGA |
| AGGTG | CACCT | C | G |
| GTACC | GGTAC | G | C |
| CGCTT | AAGCG | AGG | CCT |
| AACCG | CGGTT | TAC | GTA |
| ACTTC | GAAGT | C | G |
| TCGGT | ACCGA | G | C |
| CCTAG | CTAGG | TCG | CGA |
| CATCC | GGATG | AGC | GCT |
| TCATG | CATGA | C | G |
| ATGCA | TGCAT | G | C |
| GGAAT | ATTCC | TAG | CTA |
| TTGAC | GTCAA | ACC | GGT |

**Table 4 (scheme 4)**

| **UID** | **rc UID** | **i5 punc** | **i7 punc** |
|---|---|---|---|
| AA | TT | TCC | GGA |
| AC | GT | C | G |
| AG | CT | AAG | CTT |
| AT | AT | G | C |
| CA | TG | G | C |
| CC | GG | AGG | CCT |
| CG | CG | C | G |
| CT | AG | TAC | GTA |
| GA | TC | AGC | GCT |
| GC | GC | G | C |
| GG | CC | TCG | CGA |
| GT | AC | C | G |
| TA | TA | C | G |
| TC | GA | TAG | CTA |
| TG | CA | G | C |
| TT | AA | ACC | GGT |

**Table 5 (scheme 2)**

| **UID** | **rc UID** | **i5 punc** | **i7 punc** |
|---|---|---|---|
| AA | TT | C | G |
| AC | GT | G | C |
| AG | CT | AAG | CTT |
| AT | AT | TCC | GGA |
| CA | TG | C | G |
| CC | GG | G | C |
| CG | CG | AGG | CCT |
| CT | AG | TAC | GTA |
| GA | TC | C | G |
| GC | GC | G | C |
| GG | CC | TCG | CGA |
| GT | AC | AGC | GCT |
| TA | TA | C | G |
| TC | GA | G | C |
| TG | CA | TAG | CTA |
| TT | AA | ACC | GGT |

**Table 6 (scheme 3)**

| **UID** | **rc UID** | **i5 punc** | **i7 punc** |
|---|---|---|---|
| AA | TT | C | G |
| AC | GT | G | C |
| AG | CT | C | G |
| AT | AT | G | C |
| CA | TG | C | G |
| CC | GG | G | C |
| CG | CG | C | G |
| CT | AG | G | C |
| GA | TC | C | G |
| GC | GC | G | C |
| GG | CC | C | G |
| GT | AC | G | C |
| TA | TA | C | G |
| TC | GA | G | C |
| TG | CA | C | G |
| TT | AA | G | C |

Referring to the adapter schemes illustrated in Tables 3-6, the UID and punctuation mark can be combined with any suitable adapter sequence. For example, the ILLUMINA i5 and i7 adapter sequences are TCTTTCCCTACACGACGCTCTTCCGATCT (SEQ ID NO:1) and AGATCGGAAGAGCACACGTCTGAACTCCAGTCAC (SEQ ID NO: 2), respectively. The UID sequence (UID) CAGAT and the i5 punctuation mark (i5 punc) C in the first row of Table 3 can be combined with the ILLUMINA i5 adapter sequence to provide the oligo sequence TCTTTCCCTACACGACGCTCTTCCGATCTCAGATC*T (SEQ ID NO: 3), where the asterisk (*) indicates a phosphorothioate bond. Similarly, the reverse complement of the UID (rc UID) ATCTG and the i7 punctuation mark (i7 punc) G (the reverse complement of the i5 punctuation mark C) can be combined with the ILLUMINA i7 adapter sequence to provide the oligo sequence GATCTGAGATCGGAAGAGCACACGTCTGAACTCCAGTCAC (SEQ ID NO: 4), where the sequence includes a 5'-phosphate group. Each of Tables 3-6 lists a set of 16 different UID/punctuation mark combinations that can be used to prepare a set of 16 oligonucleotide pairs.

For preparation of adapters, each of the oligonucleotide pairs is synthesized, purified, and annealed to provide a homogenous population of annealed adapters. Then the 16 different pools of annealed adapters are combined to make one adapter pool with 16 different UIDs. It will be appreciated that pools of adapters with more or less than 16 different UIDs can also be prepared using the described approach.

In another aspect of the present disclosure, instead of an SID on only one sequencing read, an SID can be incorporated into one or both PCR primers for amplification of products resulting from ligation of target nucleic acids with annealed adapters having different UIDs. By using primers having SIDs incorporated therein, both index reads will resulting from sequencing will provide SIDs. Within one primer pair, the SIDs can be designed to have a one-to-one mapping such that when an SID from one index read is known, the SID from the other read (from the paired end) is predictable. This one-to-one mapping of SIDs enables removal of reads in an SID when a molecule from one sample associated with a first SID attaches to a molecule from another sample associated with a second SID. In the implementation shown in Tables 7 and 8, the SIDs are the reverse of each other. One sequence is considered the 'reverse' of another sequence when the two sequences share the same sequence of nucleotides in the reverse order. For example, if a first SID has the sequence AACT, a second SID having the sequence TCAA would be the reverse of the first SID. Notably, the reverse of a sequence is different from the reverse complement of a sequence. The SIDs have a minimum pairwise edit distance of 3, so with up to 1 error, an SID can always be properly associated with the correct SID sequence. Example SIDs useful with the present disclosure are described by Faircloth and coworkers (Faircloth, et al. 2012. PLoS ONE 7(8): e42543). While the sequences in Tables 7 and 8 include 96 SID pairs, it will be appreciated that yet other sequences, combinations, and numbers of SIDs can be used in the context of the present disclosure.

**Table 7**

| **Pair** | **Forward Primer (SEQ. ID. NOs: 5-100)** |
|---|---|
| 1 | |
| 2 | |
| 3 | |
| 4 | |
| 5 | |
| 6 | |
| 7 | |
| 8 | |
| 9 | |
| 10 | |
| 11 | |
| 12 | |
| 13 | |
| 14 | |
| 15 | |
| 16 | |
| 17 | |
| 18 | |
| 19 | |
| 20 | |
| 21 | |
| 22 | |
| 23 | |
| 24 | |
| 25 | |
| 26 | |
| 27 | |
| 28 | |
| 29 | |
| 30 | |
| 31 | |
| 32 | |
| 33 | |
| 34 | |
| 35 | |
| 36 | |
| 37 | |
| 38 | |
| 39 | |
| 40 | |
| 41 | |
| 42 | |
| 43 | |
| 44 | |
| 45 | |
| 46 | |
| 47 | |
| 48 | |
| 49 | |
| 50 | |
| 51 | |
| 52 | |
| 53 | |
| 54 | |
| 55 | |
| 56 | |
| 57 | |
| 58 | |
| 59 | |
| 60 | |
| 61 | |
| 62 | |
| 63 | |
| 64 | |
| 65 | |
| 66 | |
| 67 | |
| 68 | |
| 69 | |
| 70 | |
| 71 | |
| 72 | |
| 73 | |
| 74 | |
| 75 | |
| 76 | |
| 77 | |
| 78 | |
| 79 | |
| 80 | |
| 81 | |
| 82 | |
| 83 | |
| 84 | |
| 85 | |
| 86 | |
| 87 | |
| 88 | |
| 89 | |
| 90 | |
| 91 | |
| 92 | |
| 93 | |
| 94 | |
| 95 | |
| 96 | |

**Table 8**

| **Pair** | **Reverse Primer (SEQ. ID. No: 1001-196)** |
|---|---|
| 1 | |
| 2 | |
| 3 | |
| 4 | |
| 5 | |
| 6 | |
| 7 | |
| 8 | |
| 9 | |
| 10 | |
| 11 | |
| 12 | |
| 13 | |
| 14 | |
| 15 | |
| 16 | |
| 17 | |
| 18 | |
| 19 | |
| 20 | |
| 21 | |
| 22 | |
| 23 | |
| 24 | |
| 25 | |
| 26 | |
| 27 | |
| 28 | |
| 29 | |
| 30 | |
| 31 | |
| 32 | |
| 33 | |
| 34 | |
| 35 | |
| 36 | |
| 37 | |
| 38 | |
| 39 | |
| 40 | |
| 41 | |
| 42 | |
| 43 | |
| 44 | |
| 45 | |
| 46 | |
| 47 | |
| 48 | |
| 49 | |
| 50 | |
| 51 | |
| 52 | |
| 53 | |
| 54 | |
| 55 | |
| 56 | |
| 57 | |
| 58 | |
| 59 | |
| 60 | |
| 61 | |
| 62 | |
| 63 | |
| 64 | |
| 65 | |
| 66 | |
| 67 | |
| 68 | |
| 69 | |
| 70 | |
| 71 | |
| 72 | |
| 73 | |
| 74 | |
| 75 | |
| 76 | |
| 77 | |
| 78 | |
| 79 | |
| 80 | |
| 81 | |
| 82 | |
| 83 | |
| 84 | |
| 85 | |
| 86 | |
| 87 | |
| 88 | |
| 89 | |
| 90 | |
| 91 | |
| 92 | |
| 93 | |
| 94 | |
| 95 | |
| 96 | |

In one aspect, it will be appreciated that embodiments of modular nucleic acid adapters may include any combination of the features described herein. In one example, the scheme illustrated in Table 5 contemplates adapters having UIDs with a length of 2 nucleotides and variable length punctuation marks, whereas the scheme illustrated in Table 6 contemplates adapters having UIDs with a length of 2 nucleotides and single nucleotide punctuation marks (i.e., the punctuation marks are not of a variable lengths).

## Claims

1. A kit for preparing a library of nucleic acids having adapter sequences for sequencing, the kit comprising:
a first oligonucleotide having a first tail sequence, a first common sequence, a first unique identifier sequence, and a first variable length punctuation mark;
a second oligonucleotide having a second tail sequence, a second common sequence complimentary to the first common sequence, a second unique identifier sequence complimentary to the first unique identifier sequence , and a second variable length punctuation mark complimentary to the first variable length punctuation mark;
a first primer having a first sample identifier sequence and a first priming sequence at a 3' end of the first primer, the first priming sequence including the first tail sequence of the first oligonucleotide; and
a second primer having a second sample identifier sequence and a second priming sequence at a 3' end of the second primer, the second priming sequence being complimentary to the second tail sequence of the second oligonucleotide.

2. The kit of claim 1, wherein the first sample identifier sequence and the second sample identifier sequence have a one-to-one mapping.

3. The kit of claim 2, wherein the first variable length punctuation mark has a length of 2-4 nucleotides.

4. The kit of claim 2, where the first variable length punctuation mark includes at least one of a G and a C nucleotide.

5. The kit of claim 1, wherein the first unique identifier sequence has a length of at least 5 nucleotides.

6. The kit of claim 5, wherein the first unique identifier sequence has a pairwise edit distance of at least 3.

7. A kit for preparing a library of nucleic acids having adapter sequences for sequencing, the kit comprising:
a plurality of oligonucleotide pairs, each of the oligonucleotide pairs including:
a first oligonucleotide having a first tail sequence, a first common sequence, a first unique identifier sequence, and a first variable length punctuation mark, and
a second oligonucleotide having a second tail sequence, a second common sequence complimentary to the first common sequence, a second unique identifier sequence complimentary to the first unique identifier sequence , and a second variable length punctuation mark complimentary to the first variable length punctuation mark,
a first primer having a first sample identifier sequence and a first priming sequence at a 3' end of the first primer, the first priming sequence including the first tail sequence of the first oligonucleotide; and
a second primer having a second sample identifier sequence and a second priming sequence at a 3' end of the second primer, the second priming sequence being complimentary to the second tail sequence of the second oligonucleotide.

8. The kit of claim 7, wherein each of the first unique identifier sequences of each of the plurality of oligonucleotide pairs is different.

9. The kit of claim 7, wherein each of the first tail sequences of each of the plurality of oligonucleotide pairs is the same.

10. The kit of claim 7, wherein each of the second tail sequences of each of the plurality of oligonucleotide pairs is the same.

11. The kit of claim 7, wherein each of the plurality of oligonucleotide pairs are annealed to form a forked adapter.

12. The kit of claim 7, wherein the first sample identifier sequence and the second sample identifier sequence have a one-to-one mapping.

13. The kit of claim 7, wherein each of the first unique identifier sequences has a length of at least 5 nucleotides.

14. The kit of claim 15, wherein each of the first unique identifier sequences has a pairwise edit distance of at least 3.

15. A method of preparing a library of nucleic acid molecules, the method comprising:
attaching one of a plurality of oligonucleotide adapters to each end of a target nucleic acid to provide an adapter-target-adapter construct, each of the plurality of oligonucleotide adapters having:
a first oligonucleotide having a first tail sequence, a first common sequence, a first unique identifier sequence, and a first variable length punctuation mark, and
a second oligonucleotide having a second tail sequence, a second common sequence complimentary to the first common sequence, a second unique identifier sequence complimentary to the first unique identifier sequence , and a second variable length punctuation mark complimentary to the first variable length punctuation mark;
annealing a first primer to the adapter-target-adapter construct, the first primer having a first sample identifier sequence and a first priming sequence at a 3' end of the first primer, the first priming sequence including the first tail sequence of the first oligonucleotide; and
extending each of the first primer and the second primer to form extension products complementary to each strand of the adapter-target-adapter constructs.

## Patentansprüche

1. Kit zum Erstellen einer Bibliothek von Nukleinsäuren mit Adaptersequenzen zum Sequenzieren, wobei der Kit Folgendes umfasst:
ein erstes Oligonukleotid mit einer ersten Schwanzsequenz, einer ersten gemeinsamen Sequenz, einer ersten nur einmal vorhandenen Identifikationssequenz und einem ersten Satzzeichen variabler Länge;
ein zweites Oligonukleotid mit einer zweiten Schwanzsequenz, einer zweiten gemeinsamen Sequenz, die zu der ersten gemeinsamen Sequenz komplementär ist, einer zweiten nur einmal vorhandenen Identifikationssequenz, die zu der ersten nur einmal vorhandenen Identifikationssequenz komplementär ist, und einem zweiten Satzzeichen variabler Länge, das zu dem ersten Satzzeichen variabler Länge komplementär ist;
einen ersten Primer mit einer ersten Probenidentifikationssequenz und einer ersten Primingsequenz an einem 3'-Ende des ersten Primers, wobei die erste Primingsequenz die erste Schwanzsequenz des ersten Oligonukleotids einschließt; und
einen zweiten Primer mit einer zweiten Probenidentifikationssequenz und einer zweiten Primingsequenz an einem 3'-Ende des zweiten Primers, wobei die zweite Primingsequenz zu der zweiten Schwanzsequenz des zweiten Oligonukleotids komplementär ist.

2. Kit nach Anspruch 1, wobei die erste Probenidentifikationssequenz und die zweite Probenidentifikationssequenz eine Eins-zu-eins-Kartierung aufweisen.

3. Kit nach Anspruch 2, wobei das erste Satzzeichen variabler Länge eine Länge von 2 - 4 Nukleotiden hat.

4. Kit nach Anspruch 2, wobei das erste Satzzeichen variabler Länge mindestens eines von einem G- und einem C-Nukleotid einschließt.

5. Kit nach Anspruch 1, wobei die erste nur einmal vorhandene Identifikationssequenz eine Länge von mindestens 5 Nukleotiden hat.

6. Kit nach Anspruch 5, wobei die erste nur einmal vorhandene Identifikationssequenz einen paarweisen Editierabstand von mindestens 3 aufweist.

7. Kit zum Erstellen einer Bibliothek von Nukleinsäuren mit Adaptersequenzen zum Sequenzieren, wobei der Kit Folgenden umfasst:
eine Vielzahl von Oligonukleotidpaaren, wobei jedes der Oligonukleotidpaare Folgendes einschließt:
ein erstes Oligonukleotid mit einer ersten Schwanzsequenz, einer ersten gemeinsamen Sequenz, einer ersten nur einmal vorhandenen Identifikationssequenz und einem ersten Satzzeichen variabler Länge und
ein zweites Oligonukleotid mit einer zweiten Schwanzsequenz, einer zweiten gemeinsamen Sequenz, die zu der ersten gemeinsamen Sequenz komplementär ist, einer zweiten nur einmal vorhandenen Identifikationssequenz, die zu der ersten nur einmal vorhandenen Identifikationssequenz komplementär ist, und einem zweiten Satzzeichen variabler Länge, das zu dem ersten Satzzeichen variabler Länge komplementär ist,
einen ersten Primer mit einer ersten Probenidentifikationssequenz und einer ersten Primingsequenz an einem 3'-Ende des ersten Primers, wobei die erste Primingsequenz die erste Schwanzsequenz des ersten Oligonukleotids einschließt; und
einen zweiten Primer mit einer zweiten Probenidentifikationssequenz und einer zweiten Primingsequenz an einem 3'-Ende des zweiten Primers, wobei die zweite Primingsequenz zu der zweiten Schwanzsequenz des zweiten Oligonukleotids komplementär ist.

8. Kit nach Anspruch 7, wobei jede von den ersten nur einmal vorhandenen Identifikationssequenzen von jedem der Vielzahl von Oligonukleotidpaaren verschieden ist.

9. Kit nach Anspruch 7, wobei jede der ersten Schwanzsequenzen von jedem der Vielzahl von Oligonukleotidpaaren dieselbe ist.

10. Kit nach Anspruch 7, wobei jede der zweiten Schwanzsequenzen von jedem der Vielzahl von Oligonukleotidpaaren dieselbe ist.

11. Kit nach Anspruch 7, wobei jedes der Vielzahl von Oligonukleotidpaaren unter Bildung eines gegabelten Adapters annealed wird.

12. Kit nach Anspruch 7, wobei die erste Probenidentifikationssequenz und die zweite Probenidentifikationssequenz eine Eins-zu-eins-Kartierung aufweisen.

13. Kit nach Anspruch 7, wobei jede von den ersten nur einmal vorhandenen Identifikationssequenzen eine Länge von mindestens 5 Nukleotiden hat.

14. Kit nach Anspruch 15, wobei jede von den ersten nur einmal vorhandenen Identifikationssequenzen einen paarweisen Editierabstand von mindestens 3 hat.

15. Verfahren zum Erstellen einer Bibliothek von Nukleinsäuremolekülen, wobei das Verfahren Folgendes umfasst:
Anbringen eines von einer Vielzahl von Oligonukleotidadaptern an jedes Ende einer Zielnukleinsäure zum Bereitstellen eines Adapter-Ziel-Adapter-Konstruktes, wobei jeder der Vielzahl von Oligonukleotidadaptern Folgendes aufweist:
ein erstes Oligonukleotid mit einer ersten Schwanzsequenz, einer ersten gemeinsamen Sequenz, einer ersten nur einmal vorhandenen Identifikationssequenz und einem ersten Satzzeichen variabler Länge und
ein zweites Oligonukleotid mit einer zweiten Schwanzsequenz, einer zweiten gemeinsamen Sequenz, die zu der ersten gemeinsamen Sequenz komplementär ist, einer zweiten nur einmal vorhandenen Identifikationssequenz, die zu der ersten nur einmal vorhandenen Identifikationssequenz komplementär ist, und einem zweiten Satzzeichen variabler Länge, das zu dem ersten Satzzeichen variabler Länge komplementär ist;
Annealen eines ersten Primers an das Adapter-Ziel-Adapter-Konstrukt, wobei der erste Primer eine erste Probenidentifikationssequenz und eine erste Primingsequenz an einem 3'-Ende des ersten Primers aufweist, wobei die erste Primingsequenz die erste Schwanzsequenz des ersten Oligonukleotids einschließt; und
Verlängern jedes von dem ersten Primer und dem zweiten Primer unter Bildung von Verlängerungsprodukten, die zu jedem Strang der Adapter-Ziel-Adapter-Konstrukte komplementär sind.

## Revendications

1. Kit de préparation d'une banque d'acides nucléiques ayant des séquences d'adaptateurs pour le séquençage, le kit comprenant :
un premier oligonucléotide ayant une première séquence de queue, une première séquence commune, une première séquence d'identification unique et une première marque de ponctuation de longueur variable ;
un second oligonucléotide ayant une seconde séquence de queue, une seconde séquence commune complémentaire de la première séquence commune, une seconde séquence d'identification unique complémentaire de la première séquence d'identification unique et une seconde marque de ponctuation de longueur variable complémentaire de la première marque de ponctuation de longueur variable ;
une première amorce ayant une première séquence d'identification d'échantillon et une première séquence d'amorçage au niveau d'une extrémité 3' de la première amorce, la première séquence d'amorçage comprenant la première séquence de queue du premier oligonucléotide ; et
une seconde amorce ayant une seconde séquence d'identification d'échantillon et une seconde séquence d'amorçage au niveau d'une extrémité 3' de la seconde amorce, la seconde séquence d'amorçage étant complémentaire de la seconde séquence de queue du second oligonucléotide.

2. Kit selon la revendication 1, dans lequel la première séquence d'identification d'échantillon et la seconde séquence d'identification d'échantillon ont un mappage biunivoque.

3. Kit selon la revendication 2, dans lequel la première marque de ponctuation de longueur variable a une longueur de 2 à 4 nucléotides.

4. Kit selon la revendication 2, où la première marque de ponctuation de longueur variable comprend au moins un parmi un nucléotide G et un nucléotide C.

5. Kit selon la revendication 1, dans lequel la première séquence d'identification unique a une longueur d'au moins 5 nucléotides.

6. Kit selon la revendication 5, dans lequel la première séquence d'identification unique a une distance d'édition par paire d'au moins 3.

7. Kit de préparation d'une banque d'acides nucléiques ayant des séquences d'adaptateurs pour le séquençage, le kit comprenant :
une pluralité de paires d'oligonucléotides, chacune des paires d'oligonucléotides comprenant :
un premier oligonucléotide ayant une première séquence de queue, une première séquence commune, une première séquence d'identification unique et une première marque de ponctuation de longueur variable, et
un second oligonucléotide ayant une seconde séquence de queue, une seconde séquence commune complémentaire de la première séquence commune, une seconde séquence d'identification unique complémentaire de la première séquence d'identification unique et une seconde marque de ponctuation de longueur variable complémentaire de la première marque de ponctuation de longueur variable,
une première amorce ayant une première séquence d'identification d'échantillon et une première séquence d'amorçage au niveau d'une extrémité 3' de la première amorce, la première séquence d'amorçage comprenant la première séquence de queue du premier oligonucléotide ; et
une seconde amorce ayant une seconde séquence d'identification d'échantillon et une seconde séquence d'amorçage au niveau d'une extrémité 3' de la seconde amorce, la seconde séquence d'amorçage étant complémentaire de la seconde séquence de queue du second oligonucléotide.

8. Kit selon la revendication 7, dans lequel chacune des premières séquences d'identification uniques de chacune de la pluralité de paires d'oligonucléotides est différente.

9. Kit selon la revendication 7, dans lequel chacune des premières séquences de queue de chacune de la pluralité de paires d'oligonucléotides est identique.

10. Kit selon la revendication 7, dans lequel chacune des secondes séquences de queue de chacune de la pluralité de paires d'oligonucléotides est identique.

11. Kit selon la revendication 7, dans lequel chacune de la pluralité de paires d'oligonucléotides sont hybridées pour former un adaptateur en forme de fourche.

12. Kit selon la revendication 7, dans lequel la première séquence d'identification d'échantillon et la seconde séquence d'identification d'échantillon ont un mappage biunivoque.

13. Kit selon la revendication 7, dans lequel chacune des premières séquences d'identification uniques a une longueur d'au moins 5 nucléotides.

14. Kit selon la revendication 15, dans lequel chacune des premières séquences d'identification uniques a une distance d'édition par paire d'au moins 3.

15. Procédé de préparation d'une banque de molécules d'acides nucléiques, le procédé comprenant :
la fixation d'un parmi une pluralité d'adaptateurs oligonucléotidiques à chaque extrémité d'un acide nucléique cible pour fournir une construction adaptateur-cible-adaptateur, chacun de la pluralité d'adaptateurs oligonucléotidiques ayant :
un premier oligonucléotide ayant une première séquence de queue, une première séquence commune, une première séquence d'identification unique et une première marque de ponctuation de longueur variable, et
un second oligonucléotide ayant une seconde séquence de queue, une seconde séquence commune complémentaire de la première séquence commune, une seconde séquence d'identification unique complémentaire de la première séquence d'identification unique et une seconde marque de ponctuation de longueur variable complémentaire de la première marque de ponctuation de longueur variable ;
l'hybridation d'une première amorce à la construction adaptateur-cible-adaptateur, la première amorce ayant une première séquence d'identification d'échantillon et une première séquence d'amorçage au niveau d'une extrémité 3' de la première amorce, la première séquence d'amorçage comprenant la première séquence de queue du premier oligonucléotide ; et
l'extension de chacune de la première amorce et de la seconde amorce pour former des produits d'extension complémentaires de chaque brin des constructions adaptateur-cible-adaptateur.
